# EUROPEAN PATENT APPLICATION

(11) **EP 0 841 399 A2**
(43) Date of publication of application: **13.05.1998**
(21) Application number: 97309003.8
(22) Date of filing: 10.11.1997
(51) Int. Cl.: C12N 15/57, C12N 9/64, C12N 5/10, C07K 16/40, C12N 15/11, A61K 38/48, A61K 48/00, A61K 39/395, A61K 31/70, C12Q 1/68, G01N 33/68

(54) **Mammalian fin-1 nucleic acid (a caspase) and protein sequences and uses therefor**

(30) Priority: 12.11.1996 US 748086
(71) Applicant: SMITHKLINE BEECHAM CORPORATION, Philadelphia Pennsylvania 19103 (US)
(72) Inventor: Kikly, Kristine, SmithKline Beecham Pharm., King of Prussia, Pennsylvania 19406 (US); Emery, John G., SmithKline Beecham Pharm., King of Prussia, Pennsylvania 19406 (US)
(74) Representative: Crump, Julian Richard John

(57) **Abstract**

Human FIN-1 polypeptides and DNA (RNA) encoding such protein and a procedure for producing such polypeptides by recombinant techniques is disclosed. Also disclosed are methods for utilizing such human FIN-1 in the development of treatments for viral infections and cancers, particularly those associated with solid tumors, among others, are also disclosed. Also disclosed are diagnostic assays for detecting diseases related to mutations in the nucleic acid sequences and altered concentrations of the polypeptides.

SEQUENCE LISTING

(1) GENERAL INFORMATION:

(i) APPLICANT: SmithKline Beecham Corporation

(ii) TITLE OF INVENTION: Mammalian FIN-1 Nucleic Acid and Protein
Sequence and Uses Therefor

(iii) NUMBER OF SEQUENCES: 15

(iv) CORRESPONDENCE ADDRESS:
(A) ADDRESSEE: F J Cleveland & Company
(B) STREET: 40/43 Chancery Lane
(C) CITY: London
(D) COUNTY: -
(E) COUNTRY: United Kingdom
(F) POST CODE: TW8 9EP

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US
(B) FILING DATE:
(C) CLASSIFICATION:

(viii) ATTORNEY/AGENT INFORMATION:
(A) NAME: CRUMP, Julian Richard John
(B) GENERAL AUTHORISATION NUMBER: 37127
(C) REFERENCE/DOCKET NUMBER: ATG50034

(ix) TELECOMMUNICATION INFORMATION:
(A) TELEPHONE: +44 171 405 5875
(B) TELEFAX: +44 171 831 0749

(2) INFORMATION FOR SEQ ID NO:1:

(i) SEQUENCE CHARACTERISTICS:
(A) LENGTH: 2188 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION: 422..1861

(xi) SEQUENCE DESCRIPTION: SEQ ID NO:1:

CCTCACCGAC GAGTCTCAAC TAAAAGGGAC TCCCGGAGCT AGGGGTGGGG ACTCGGCCTC 60

ACACAGTGAG TGCCGGCTAT TGGACTTTTG TCCAGTGACA GCTGAGACAA CAAGGACCAC 120

GGGAGGAGGT GTAGGAGAGA AGCGCCGCGA ACAGCGATCG CCCAGCACCA AGTCCGCTTC 180

CAGGCTTTCG GTTTCTTTGC CTCCATCTTG GGTGCGCCTT CCCGGCGTCT AGGGGAGCGA 240

AGGCTGAGGT GGCAGCGGCA GGAGAGTCCG GCCGCGACAG GACGAACTCC CCCACTGGAA 300

AGGATTCTGA AAGAAATGAA GTCAGCCCTC AGAAATGAAG TTGACTGCCT GCTGGCTTTC 360

TGTTGACTGG CCCGGAGCTG TACTGCAAGA CCCTTGTGAG CTTCCCTAGT CTAAGAGTAG 420

G ATG TCT GCT GAA GTC ATC CAT CAG GTT GAA GAA GCA CTT GAT ACA 466
Met Ser Ala Glu Val Ile His Gln Val Glu Glu Ala Leu Asp Thr
1               5                   10                  15

GAT GAG AAG GAG ATG CTG CTC TTT TTG TGC CGG GAT GTT GCT ATA GAT 514
Asp Glu Lys Glu Met Leu Leu Phe Leu Cys Arg Asp Val Ala Ile Asp
                20                  25                  30

GTG GTT CCA CCT AAT GTC AGG GAC CTT CTG GAT ATT TTA CGG GAA AGA 562
Val Val Pro Pro Asn Val Arg Asp Leu Leu Asp Ile Leu Arg Glu Arg
            35                  40                  45

GGT AAG CTG TCT GTC GGG GAC TTG GCT GAA CTG CTC TAC AGA GTG AGG 610
Gly Lys Leu Ser Val Gly Asp Leu Ala Glu Leu Leu Tyr Arg Val Arg
        50                  55                  60

CGA TTT GAC CTG CTC AAA CGT ATC TTG AAG ATG GAC AGA AAA GCT GTG 658
Arg Phe Asp Leu Leu Lys Arg Ile Leu Lys Met Asp Arg Lys Ala Val
    65                  70                  75

GAG ACC CAC CTG CTC AGG AAC CCT CAC CTT GTT TCG GAC TAT AGA GTG 706
Glu Thr His Leu Leu Arg Asn Pro His Leu Val Ser Asp Tyr Arg Val
80                  85                  90                  95

CTG ATG GCA GAG ATT GGT GAG GAT TTG GAT AAA TCT GAT GTG TCC TCA 754
Leu Met Ala Glu Ile Gly Glu Asp Leu Asp Lys Ser Asp Val Ser Ser
                100                 105                 110

TTA ATT TTC CTC ATG AAG GAT TAC ATG GGC CGA GGC AAG ATA AGC AAG 802
Leu Ile Phe Leu Met Lys Asp Tyr Met Gly Arg Gly Lys Ile Ser Lys
            115                 120                 125

GAG AAG AGT TTC TTG GAC CTT GTG GTT GAG TTG GAG AAA CTA AAT CTG 850
Glu Lys Ser Phe Leu Asp Leu Val Val Glu Leu Glu Lys Leu Asn Leu
        130                 135                 140

GTT GCC CCA GAT CAA CTG GAT TTA TTA GAA AAA TGC CTA AAG AAC ATC 898
Val Ala Pro Asp Gln Leu Asp Leu Leu Glu Lys Cys Leu Lys Asn Ile
    145                 150                 155

CAC AGA ATA GAC CTG AAG ACA AAA ATC CAG AAG TAC AAG CAG TCT GTT 946
His Arg Ile Asp Leu Lys Thr Lys Ile Gln Lys Tyr Lys Gln Ser Val
160                 165                 170                 175

CAA GGA GCA GGG ACA AGT TAC AGG AAT GTT CTC CAA GCA GCA ATC CAA 994
Gln Gly Ala Gly Thr Ser Tyr Arg Asn Val Leu Gln Ala Ala Ile Gln
                180                 185                 190

AAG AGT CTC AAG GAT CCT TCA AAT AAC TTC AGG CTC CAT AAT GGG AGA 1042
Lys Ser Leu Lys Asp Pro Ser Asn Asn Phe Arg Leu His Asn Gly Arg
            195                 200                 205

AGT AAA GAA CAA AGA CTT AAG GAA CAG CTT GGC GCT CAA CAA GAA CCA 1090
Ser Lys Glu Gln Arg Leu Lys Glu Gln Leu Gly Ala Gln Gln Glu Pro
        210                 215                 220

GTG AAG AAA TCC ATT CAG GAA TCA GAA GCT TTT TTG CCT CAG AGC ATA 1138
Val Lys Lys Ser Ile Gln Glu Ser Glu Ala Phe Leu Pro Gln Ser Ile
    225                 230                 235

CCT GAA GAG AGA TAC AAG ATG AAG AGC AAG CCC CTA GGA ATC TGC CTG 1186
Pro Glu Glu Arg Tyr Lys Met Lys Ser Lys Pro Leu Gly Ile Cys Leu
240                 245                 250                 255

ATA ATC GAT TGC ATT GGC AAT GAG ACA GAG CTT CTT CGA GAC ACC TTC 1234
Ile Ile Asp Cys Ile Gly Asn Glu Thr Glu Leu Leu Arg Asp Thr Phe
                260                 265                 270

ACT TCC CTG GGC TAT GAA GTC CAG AAA TTC TTG CAT CTC AGT ATG CAT 1282
Thr Ser Leu Gly Tyr Glu Val Gln Lys Phe Leu His Leu Ser Met His
            275                 280                 285

GGT ATA TCC CAG ATT CTT GGC CAA TTT GCC TGT ATG CCC GAG CAC CGA 1330
Gly Ile Ser Gln Ile Leu Gly Gln Phe Ala Cys Met Pro Glu His Arg
        290                 295                 300

GAC TAC GAC AGC TTT GTG TGT GTC CTG GTG AGC CGA GGA GGC TCC CAG 1378
Asp Tyr Asp Ser Phe Val Cys Val Leu Val Ser Arg Gly Gly Ser Gln
    305                 310                 315

AGT GTG TAT GGT GTG GAT CAG ACT CAC TCA GGG CTC CCC CTG CAT CAC 1426
Ser Val Tyr Gly Val Asp Gln Thr His Ser Gly Leu Pro Leu His His
320                 325                 330                 335

ATC AGG AGG ATG TTC ATG GGA GAT TCA TGC CCT TAT CTA GCA GGG AAG 1474
Ile Arg Arg Met Phe Met Gly Asp Ser Cys Pro Tyr Leu Ala Gly Lys
                340                 345                 350

CCA AAG ATG TTT TTT ATT CAG AAC TAT GTG GTG TCA GAG GGC CAG CTG 1522
Pro Lys Met Phe Phe Ile Gln Asn Tyr Val Val Ser Glu Gly Gln Leu
            355                 360                 365

GAG GAC AGC AGC CTC TTG GAG GTG GAT GGG CCA GCG ATG AAG AAT GTG 1570
Glu Asp Ser Ser Leu Leu Glu Val Asp Gly Pro Ala Met Lys Asn Val
        370                 375                 380

GAA TTC AAG GCT CAG AAG CGA GGG CTG TGC ACA GTT CAC CGA GAA GCT 1618
Glu Phe Lys Ala Gln Lys Arg Gly Leu Cys Thr Val His Arg Glu Ala
    385                 390                 395

GAC TTC TTC TGG AGC CTG TGT ACT GCG GAC ATG TCC CTG CTG GAG CAG 1666
Asp Phe Phe Trp Ser Leu Cys Thr Ala Asp Met Ser Leu Leu Glu Gln
400                 405                 410                 415

TCT CAC AGC TCA CCG TCC CTG TAC CTG CAG TGC CTC TCC CAG AAA CTG 1714
Ser His Ser Ser Pro Ser Leu Tyr Leu Gln Cys Leu Ser Gln Lys Leu
                420                 425                 430

AGA CAA GAA AGA AAA CGC CCA CTC CTG GAT CTT CAC ATT GAA CTC AAT 1762
Arg Gln Glu Arg Lys Arg Pro Leu Leu Asp Leu His Ile Glu Leu Asn
            435                 440                 445

GGC TAC ATG TAT GAT TGG AAC AGC AGA GTT TCT GCC AAG GAG AAA TAT 1810
Gly Tyr Met Tyr Asp Trp Asn Ser Arg Val Ser Ala Lys Glu Lys Tyr
        450                 455                 460

TAT GTC TGG CTG CAG CAC ACT CTG AGA AAG AAA CTT ATC CTC TCC TAC 1858
Tyr Val Trp Leu Gln His Thr Leu Arg Lys Lys Leu Ile Leu Ser Tyr
    465                 470                 475

ACA TAAGAAACCA AAAGGCTGGG CGTAGTGGCT CACACCTGTA ATCCCAGCAC 1911
Thr
480

TTTGGGAGGC CAAGGAGGGC AGATCACTTC AGGTCAGGAG TTCGAGACCA GCCTGGCCAA 1971

CATGGTAAAC GCTGTCCCTA GTAAAAATAC AAAAATTAGC TGGGTGTGGG TGTGGGTACC 2031

TGTATTCCCA GTTACTTGGG AGGCTGAGGT GGGAGGATCT TTTGAACCCA GGAGTTCAGG 2091

GTCATAGCAT GCTGTGATTG TGCCTACGAA TAGCCACTGC ATACCAACCT GGGCAATATA 2151

GCAAGATCCC ATCTCTTTAA AAAAAAAAAA AAAAAAA 2188

(2) INFORMATION FOR SEQ ID NO:2:

(i) SEQUENCE CHARACTERISTICS:
(A) LENGTH: 480 amino acids
(B) TYPE: amino acid
(D) TOPOLOGY: linear

(ii) MOLECULE TYPE: protein

(xi) SEQUENCE DESCRIPTION: SEQ ID NO:2:

Met Ser Ala Glu Val Ile His Gln Val Glu Glu Ala Leu Asp Thr Asp
1               5                   10                  15

Glu Lys Glu Met Leu Leu Phe Leu Cys Arg Asp Val Ala Ile Asp Val
            20                  25                  30

Val Pro Pro Asn Val Arg Asp Leu Leu Asp Ile Leu Arg Glu Arg Gly
        35                  40                  45

Lys Leu Ser Val Gly Asp Leu Ala Glu Leu Leu Tyr Arg Val Arg Arg
    50                  55                  60

Phe Asp Leu Leu Lys Arg Ile Leu Lys Met Asp Arg Lys Ala Val Glu
65                  70                  75                  80

Thr His Leu Leu Arg Asn Pro His Leu Val Ser Asp Tyr Arg Val Leu
                85                  90                  95

Met Ala Glu Ile Gly Glu Asp Leu Asp Lys Ser Asp Val Ser Ser Leu
            100                 105                 110

Ile Phe Leu Met Lys Asp Tyr Met Gly Arg Gly Lys Ile Ser Lys Glu
        115                 120                 125

Lys Ser Phe Leu Asp Leu Val Val Glu Leu Glu Lys Leu Asn Leu Val
    130                 135                 140

Ala Pro Asp Gln Leu Asp Leu Leu Glu Lys Cys Leu Lys Asn Ile His
145                 150                 155                 160

Arg Ile Asp Leu Lys Thr Lys Ile Gln Lys Tyr Lys Gln Ser Val Gln
                165                 170                 175

Gly Ala Gly Thr Ser Tyr Arg Asn Val Leu Gln Ala Ala Ile Gln Lys
            180                 185                 190

Ser Leu Lys Asp Pro Ser Asn Asn Phe Arg Leu His Asn Gly Arg Ser
        195                 200                 205

Lys Glu Gln Arg Leu Lys Glu Gln Leu Gly Ala Gln Gln Glu Pro Val
    210                 215                 220

Lys Lys Ser Ile Gln Glu Ser Glu Ala Phe Leu Pro Gln Ser Ile Pro
225                 230                 235                 240

Glu Glu Arg Tyr Lys Met Lys Ser Lys Pro Leu Gly Ile Cys Leu Ile
                245                 250                 255

Ile Asp Cys Ile Gly Asn Glu Thr Glu Leu Leu Arg Asp Thr Phe Thr
            260                 265                 270

Ser Leu Gly Tyr Glu Val Gln Lys Phe Leu His Leu Ser Met His Gly
        275                 280                 285

Ile Ser Gln Ile Leu Gly Gln Phe Ala Cys Met Pro Glu His Arg Asp
    290                 295                 300

Tyr Asp Ser Phe Val Cys Val Leu Val Ser Arg Gly Gly Ser Gln Ser
305                 310                 315                 320

Val Tyr Gly Val Asp Gln Thr His Ser Gly Leu Pro Leu His His Ile
                325                 330                 335

Arg Arg Met Phe Met Gly Asp Ser Cys Pro Tyr Leu Ala Gly Lys Pro
            340                 345                 350

Lys Met Phe Phe Ile Gln Asn Tyr Val Val Ser Glu Gly Gln Leu Glu
        355                 360                 365

Asp Ser Ser Leu Leu Glu Val Asp Gly Pro Ala Met Lys Asn Val Glu
    370                 375                 380

Phe Lys Ala Gln Lys Arg Gly Leu Cys Thr Val His Arg Glu Ala Asp
385                 390                 395                 400

Phe Phe Trp Ser Leu Cys Thr Ala Asp Met Ser Leu Leu Glu Gln Ser
                405                 410                 415

His Ser Ser Pro Ser Leu Tyr Leu Gln Cys Leu Ser Gln Lys Leu Arg
            420                 425                 430

Gln Glu Arg Lys Arg Pro Leu Leu Asp Leu His Ile Glu Leu Asn Gly
        435                 440                 445

Tyr Met Tyr Asp Trp Asn Ser Arg Val Ser Ala Lys Glu Lys Tyr Tyr
    450                 455                 460

Val Trp Leu Gln His Thr Leu Arg Lys Lys Leu Ile Leu Ser Tyr Thr
465                 470                 475                 480

(2) INFORMATION FOR SEQ ID NO:3:

     (i) SEQUENCE CHARACTERISTICS:
          (A) LENGTH: 277 amino acids
          (B) TYPE: amino acid
          (C) STRANDEDNESS:
          (D) TOPOLOGY: linear

    (ii) MOLECULE TYPE: protein

    (xi) SEQUENCE DESCRIPTION: SEQ ID NO:3:

     Met Glu Asn Asn Glu Thr Ser Val Asp Ser Lys Ser Ile Lys Asn Phe
     1               5                   10                  15

     Glu Val Lys Thr Ile His Gly Ser Lys Ser Met Asp Ser Gly Ile Tyr
                 20                  25                  30

     Leu Asp Ser Ser Tyr Lys Met Asp Tyr Pro Glu Met Gly Val Cys Ile
             35                  40                  45

     Ile Ile Asn Asn Lys Asn Phe His Lys Ser Thr Gly Met Thr Pro Arg
         50                  55                  60

     Ser Gly Thr Asp Val Asp Ala Ala Lys Leu Arg Glu Thr Phe Met Ala
     65                  70                  75                  80

     Leu Lys Tyr Glu Val Arg Asn Lys Asn Asp Leu Thr Arg Glu Glu Ile
                     85                  90                  95

     Val Glu Leu Met Lys Asn Ala Ser Lys Glu Asp His Ser Lys Arg Ser
                 100                 105                 110

     Ser Phe Val Cys Val Ile Leu Ser His Gly Asp Glu Gly Val Ile Phe
             115                 120                 125

     Gly Thr Asp Gly Pro Ile Asp Leu Lys Lys Leu Thr Ser Tyr Phe Arg
         130                 135                 140

     Gly Asp Tyr Cys Arg Ser Leu Ile Gly Lys Pro Lys Leu Phe Ile Ile
     145                 150                 155                 160

     Gln Ala Cys Arg Gly Thr Glu Leu Asp Cys Gly Ile Glu Thr Asp Ser
                     165                 170                 175

     Gly Thr Glu Asp Asp Met Thr Cys Gln Lys Ile Pro Val Glu Ala Asp
                 180                 185                 190

     Phe Leu Tyr Ala Tyr Ser Thr Ala Pro Gly Tyr Tyr Ser Trp Arg Asn
             195                 200                 205

     Pro Lys Asp Gly Ser Trp Phe Ile Gln Ser Leu Cys Ser Met Leu Lys
         210                 215                 220

Leu Tyr Ala His Lys Leu Glu Phe Met His Ile Leu Thr Arg Val Asn
225                 230                 235                 240

Arg Lys Val Ala Thr Glu Phe Glu Ser Phe Ser Leu Asp Ser Thr Phe
                245                 250                 255

His Ala Lys Lys Gln Ile Pro Cys Ile Val Ser Met Leu Thr Lys Glu
            260                 265                 270

Leu Tyr Phe Tyr His
        275

(2) INFORMATION FOR SEQ ID NO:4:

    (i) SEQUENCE CHARACTERISTICS:
        (A) LENGTH: 303 amino acids
        (B) TYPE: amino acid
        (C) STRANDEDNESS:
        (D) TOPOLOGY: linear

    (ii) MOLECULE TYPE: protein

    (xi) SEQUENCE DESCRIPTION: SEQ ID NO:4:

(2) INFORMATION FOR SEQ ID NO:5:

    (i) SEQUENCE CHARACTERISTICS:
        (A) LENGTH: 302 amino acids
        (B) TYPE: amino acid
        (C) STRANDEDNESS:
        (D) TOPOLOGY: linear

    (ii) MOLECULE TYPE: protein

    (xi) SEQUENCE DESCRIPTION: SEQ ID NO:5:

Tyr Glu Glu Phe Ser Lys Glu Arg Ser Ser Ser Leu Glu Gly Ser Pro
1               5                   10                  15

Asp Glu Phe Ser Asn Gly Glu Glu Leu Cys Gly Val Met Thr Ile Ser
            20                  25                  30

Asp Ser Pro Arg Glu Gln Asp Ser Glu Ser Gln Thr Leu Asp Lys Val
        35                  40                  45

Tyr Gln Met Lys Ser Lys Pro Arg Gly Tyr Cys Leu Ile Ile Asn Asn
    50                  55                  60

His Asn Phe Ala Lys Ala Arg Glu Lys Val Pro Lys Leu His Ser Ile
65                  70                  75                  80

Arg Asp Arg Asn Gly Thr His Leu Asp Ala Gly Ala Leu Thr Thr Thr
                85                  90                  95

Phe Glu Glu Leu His Phe Glu Ile Lys Pro His Asp Asp Cys Thr Val
            100                 105                 110

Glu Gln Ile Tyr Glu Ile Trp Lys Ile Tyr Gln Leu Met Asp His Ser
        115                 120                 125

Asn Met Asp Cys Phe Ile Cys Cys Ile Leu Ser His Gly Asp Lys Gly
    130                 135                 140

Ile Ile Tyr Gly Thr Asp Gly Gln Glu Gly Pro Ile Tyr Glu Leu Thr
145                 150                 155                 160

Ser Gln Phe Thr Gly Leu Lys Cys Pro Ser Leu Ala Gly Lys Pro Lys
                165                 170                 175

Val Phe Phe Ile Gln Ala Cys Gln Gly Asp Asn Tyr Gln Lys Gly Ile
            180                 185                 190

Pro Val Glu Thr Asp Ser Glu Glu Gln Pro Tyr Leu Glu Met Asp Leu
        195                 200                 205

Ser Ser Pro Gln Thr Arg Tyr Ile Pro Asp Glu Ala Asp Phe Leu Leu
    210                 215                 220

Gly Met Ala Thr Val Asn Asn Cys Val Ser Tyr Arg Asn Pro Ala Glu
225                 230                 235                 240

Gly Thr Trp Tyr Ile Gln Ser Leu Cys Gln Ser Leu Arg Glu Arg Cys
                245                 250                 255

Pro Arg Gly Asp Asp Ile Leu Thr Ile Leu Thr Glu Val Asn Tyr Glu
            260                 265                 270

Val Ser Asn Lys Asp Asp Lys Lys Asn Met Gly Lys Gln Met Pro Gln
        275                 280                 285

Pro Thr Phe Thr Leu Arg Lys Lys Leu Val Phe Pro Ser Asp
    290                 295                 300

(2) INFORMATION FOR SEQ ID NO:6:

    (i) SEQUENCE CHARACTERISTICS:
        (A) LENGTH: 292 amino acids
        (B) TYPE: amino acid
        (C) STRANDEDNESS:
        (D) TOPOLOGY: linear

    (ii) MOLECULE TYPE: protein

    (xi) SEQUENCE DESCRIPTION: SEQ ID NO:6:

Tyr Lys Arg Glu Lys Ala Ile Gln Ile Val Thr Pro Pro Val Asp Lys
1               5                   10                  15

Glu Ala Glu Ser Tyr Gln Gly Glu Glu Glu Leu Val Ser Gln Thr Asp
            20                  25                  30

Val Lys Thr Phe Leu Glu Ala Leu Pro Arg Ala Ala Val Tyr Arg Met
        35                  40                  45

Asn Arg Asn His Arg Gly Leu Cys Val Ile Val Asn Asn His Ser Phe
    50                  55                  60

Thr Ser Leu Lys Asp Arg Gln Gly Thr His Lys Asp Ala Glu Ile Leu
65                  70                  75                  80

Ser His Val Phe Gln Trp Leu Gly Phe Thr Val His Ile His Asn Asn
                85                  90                  95

Val Thr Lys Val Glu Met Glu Met Val Leu Gln Lys Gln Lys Cys Asn
            100                 105                 110

Pro Ala His Ala Asp Gly Asp Cys Phe Val Phe Cys Ile Leu Thr His
        115                 120                 125

Gly Arg Phe Gly Ala Val Tyr Ser Ser Asp Glu Ala Leu Ile Pro Ile
    130                 135                 140

Arg Glu Ile Met Ser His Phe Thr Ala Leu Gln Cys Pro Arg Leu Ala
145                 150                 155                 160

Glu Lys Pro Lys Leu Phe Phe Ile Gln Ala Cys Gln Gly Glu Glu Ile
                165                 170                 175

Gln Pro Ser Val Ser Ile Glu Ala Asp Ala Leu Asn Pro Glu Gln Ala
            180                 185                 190

Pro Thr Ser Leu Gln Asp Ser Ile Pro Ala Glu Ala Asp Phe Leu Leu
        195                 200                 205

Gly Leu Ala Thr Val Pro Gly Tyr Val Ser Phe Arg His Val Glu Glu
    210                 215                 220

Gly Ser Trp Tyr Ile Gln Ser Leu Cys Asn His Leu Lys Lys Leu Val
225                 230                 235                 240

Pro Arg His Glu Asp Ile Leu Ser Ile Leu Thr Ala Val Asn Asp Asp
                245                 250                 255

Val Ser Arg Arg Val Asp Lys Gln Gly Thr Lys Lys Gln Met Pro Gln
            260                 265                 270

Pro Ala Phe Thr Leu Arg Lys Lys Leu Val Phe Pro Val Pro Leu Asp
        275                 280                 285

Ala Leu Ser Ile
    290

(2) INFORMATION FOR SEQ ID NO:7:

    (i) SEQUENCE CHARACTERISTICS:
        (A) LENGTH: 81 amino acids
        (B) TYPE: amino acid
        (C) STRANDEDNESS:
        (D) TOPOLOGY: linear

    (ii) MOLECULE TYPE: protein

(xi) SEQUENCE DESCRIPTION: SEQ ID NO:7:

Met Asp Pro Phe Leu Val Leu Leu His Ser Val Ser Ser Ser Leu Ser
1               5                   10                  15

Ser Ser Glu Leu Thr Glu Leu Lys Phe Leu Cys Leu Gly Arg Val Gly
            20                  25                  30

Lys Arg Lys Leu Glu Arg Val Gln Ser Gly Leu Asp Leu Phe Ser Met
        35                  40                  45

Leu Leu Glu Gln Asn Asp Leu Glu Pro Gly His Thr Glu Leu Leu Arg
    50                  55                  60

Glu Leu Leu Ala Ser Leu Arg Arg His Asp Leu Leu Arg Arg Val Asp
65                  70                  75                  80

Asp

(2) INFORMATION FOR SEQ ID NO:8:

(i) SEQUENCE CHARACTERISTICS:
(A) LENGTH: 80 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO:8:

Met Asp Phe Ser Arg Asn Leu Tyr Asp Ile Gly Glu Gln Leu Asp Ser
1               5                   10                  15

Glu Asp Leu Ala Ser Leu Lys Phe Leu Ser Leu Asp Tyr Ile Pro Gln
            20                  25                  30

Arg Lys Gln Glu Pro Ile Lys Asp Ala Leu Met Leu Phe Gln Arg Leu
        35                  40                  45

Gln Glu Lys Arg Met Leu Glu Glu Ser Asn Leu Ser Phe Leu Lys Glu
    50                  55                  60

Leu Leu Phe Arg Ile Asn Arg Leu Asp Leu Leu Ile Thr Tyr Leu Asn
65                  70                  75                  80

(2) INFORMATION FOR SEQ ID NO:9:

(i) SEQUENCE CHARACTERISTICS:
(A) LENGTH: 77 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO:9:

Tyr Arg Val Met Leu Tyr Gln Ile Ser Glu Glu Val Ser Arg Ser Glu
1               5                   10                  15

Leu Arg Ser Phe Lys Phe Leu Leu Gln Glu Glu Ile Ser Lys Cys Lys
            20                  25                  30

Leu Asp Asp Asp Met Asn Leu Leu Asp Ile Phe Ile Glu Met Glu Lys
        35                  40                  45

Arg Val Ile Leu Gly Glu Gly Lys Leu Asp Ile Leu Lys Arg Val Cys
    50                  55                  60

Ala Gln Ile Asn Lys Ser Leu Leu Lys Ile Ile Asn Asp
65                  70                  75

(2) INFORMATION FOR SEQ ID NO:10:

(i) SEQUENCE CHARACTERISTICS:
(A) LENGTH: 78 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO:10:

Val Ser Phe Arg Glu Lys Leu Leu Ile Ile Asp Ser Asn Leu Gly Val
1               5                   10                  15

Gln Asp Val Glu Asn Leu Lys Phe Leu Cys Ile Gly Leu Val Pro Asn
            20                  25                  30

Lys Lys Leu Glu Lys Ser Ser Ser Ala Ser Asp Val Phe Glu His Leu
        35                  40                  45

Leu Ala Glu Asp Leu Leu Ser Glu Glu Asp Pro Phe Phe Leu Ala Glu
    50                  55                  60

Leu Leu Tyr Ile Ile Arg Gln Lys Lys Leu Leu Gln His Leu
65                  70                  75

(2) INFORMATION FOR SEQ ID NO:11:

(i) SEQUENCE CHARACTERISTICS:
(A) LENGTH: 78 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO:11:

Val Ser Leu Phe Arg Asn Leu Leu Tyr Glu Leu Ser Glu Gly Ile Asp
1               5                   10                  15

Ser Glu Asn Leu Lys Asp Met Ile Phe Leu Leu Lys Asp Ser Leu Pro
            20                  25                  30

Lys Thr Glu Met Thr Ser Leu Ser Phe Leu Ala Phe Leu Glu Lys Gln
        35                  40                  45

Gly Lys Ile Asp Glu Asp Asn Leu Thr Cys Leu Glu Asp Leu Cys Lys
    50                  55                  60

Thr Val Val Pro Lys Leu Leu Arg Asn Ile Glu Lys Tyr Lys
65                  70                  75

(2) INFORMATION FOR SEQ ID NO:12:

(i) SEQUENCE CHARACTERISTICS:
(A) LENGTH: 81 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO:12:

Met Val Glu Tyr Gly Thr Leu Phe Gln Asp Leu Thr Asn Asn Ile Thr
1               5                   10                  15

Leu Glu Asp Leu Glu Gln Leu Lys Ser Ala Cys Lys Glu Asp Ile Pro
            20                  25                  30

Ser Glu Lys Ser Glu Glu Ile Thr Thr Gly Ser Ala Trp Phe Ser Phe
        35                  40                  45

Leu Glu Ser His Asn Lys Leu Asp Lys Asp Asn Leu Ser Ile Ile Glu
    50                  55                  60

His Ile Phe Glu Ile Ser Arg Arg Pro Asp Leu Leu Thr Met Val Val
65                  70                  75                  80

Asp

(2) INFORMATION FOR SEQ ID NO:13:

(i) SEQUENCE CHARACTERISTICS:
(A) LENGTH: 82 amino acids
(B) TYPE: amino acid
(C) STRANDEDNESS:
(D) TOPOLOGY: unknown

(ii) MOLECULE TYPE: protein

(xi) SEQUENCE DESCRIPTION: SEQ ID NO:13:

Xaa Xaa Xaa Phe Xaa Xaa Xaa Leu Xaa Xaa Ile Xaa Xaa Xaa Xaa Xaa
1               5                   10                  15

Xaa Xaa Asp Xaa Xaa Xaa Leu Xaa Phe Leu Xaa Xaa Asp Xaa Xaa Xaa
            20                  25                  30

Xaa Xaa Lys Xaa Xaa Xaa Xaa Xaa Xaa Xaa Xaa Xaa Leu Phe Xaa Xaa
        35                  40                  45

Leu Xaa Xaa Xaa Xaa Xaa Leu Xaa Xaa Xaa Xaa Xaa Xaa Xaa Leu Xaa
    50                  55                  60

Xaa Xaa Leu Xaa Xaa Ile Xaa Xaa Xaa Xaa Leu Xaa Xaa Xaa Xaa Xaa
65                  70                  75                  80

Xaa Xaa

(2) INFORMATION FOR SEQ ID NO:14:

(i) SEQUENCE CHARACTERISTICS:
(A) LENGTH: 345 amino acids
(B) TYPE: amino acid
(C) STRANDEDNESS:
(D) TOPOLOGY: unknown

(ii) MOLECULE TYPE: protein

(xi) SEQUENCE DESCRIPTION: SEQ ID NO:14:

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Glu
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Tyr Xaa Met Xaa Xaa Xaa
65                  70                  75                  80

Xaa Xaa Gly Xaa Cys Xaa Ile Ile Asn Asn Xaa Xaa Phe Xaa Xaa Xaa
                85                  90                  95

Xaa Xaa Xaa Xaa Xaa Xaa Xaa Xaa Xaa Xaa Xaa Xaa Arg Xaa Gly Thr
            100                 105                 110

Xaa Xaa Asp Ala Xaa Xaa Leu Xaa Xaa Xaa Phe Xaa Xaa Leu Xaa Phe
        115                 120                 125

Glu Val Xaa Xaa Xaa Xaa Xaa Xaa Xaa Xaa Xaa Xaa Xaa Xaa Xaa Xaa
    130                 135                 140

Leu Xaa Lys Xaa Xaa Xaa Xaa Xaa Asp His Xaa Xaa Xaa Xaa Xaa Phe
145                 150                 155                 160

Val Cys Xaa Xaa Leu Ser His Gly Xaa Xaa Xaa Xaa Ile Tyr Gly Xaa
                165                 170                 175

Asp Xaa Xaa Xaa Xaa Xaa Xaa Pro Xaa Xaa Xaa Xaa Xaa Xaa Xaa Phe
            180                 185                 190

Xaa Gly Xaa Xaa Cys Xaa Xaa Leu Xaa Xaa Lys Pro Lys Leu Phe Phe
        195                 200                 205

Ile Gln Ala Cys Xaa Gly Xaa Glu Xaa Xaa Xaa Xaa Ile Xaa Xaa Xaa
    210                 215                 220

Xaa Xaa Xaa Xaa Xaa Xaa Xaa Xaa Xaa Xaa Xaa Xaa Xaa Xaa Xaa Xaa
225                 230                 235                 240

Xaa Xaa Xaa Xaa Xaa Xaa Ile Pro Xaa Glu Ala Asp Phe Leu Leu Xaa
                245                 250                 255

Xaa Xaa Thr Xaa Xaa Xaa Tyr Xaa Ser Trp Arg Xaa Xaa Xaa Xaa Gly
            260                 265                 270

Ser Trp Tyr Ile Gln Xaa Leu Cys Xaa Xaa Leu Xaa Xaa Xaa Xaa Xaa
        275                 280                 285

Xaa Xaa Xaa Xaa Xaa Leu Xaa Ile Leu Thr Xaa Val Asn Xaa Xaa Val
    290                 295                 300

Xaa Xaa Xaa Xaa Xaa Xaa Xaa Xaa Xaa Xaa Xaa Xaa Xaa Xaa Xaa Xaa
305                 310                 315                 320

Lys Gln Xaa Pro Xaa Xaa Xaa Xaa Xaa Leu Xaa Lys Xaa Leu Xaa Phe
                325                 330                 335

Xaa Xaa Xaa Xaa Xaa Xaa Xaa Xaa Xaa
            340                 345

(2) INFORMATION FOR SEQ ID NO:15:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(ix) FEATURE:
(A) NAME/KEY: Modified-site
(B) LOCATION: 4
(D) OTHER INFORMATION: /note= "Amino acid in position 4
can be either Arg or Gln"

(xi) SEQUENCE DESCRIPTION: SEQ ID NO:15:

Gln Ala Cys Xaa Gly
1               5

## Description

### FIELD OF THE INVENTION

This invention relates to novel polynucleotides and polypeptides of the family of interleukin-1β converting enzymes, hereinafter referred to as ICE, and also, relates to the death effector domain family of protein.

### BACKGROUND OF THE INVENTION

Programmed cell death (apoptosis) plays an important role in a wide variety of physiological circumstances in essentially all complex multicellular organisms (for review see, Ellis *et al., Cell Biology,* 7:663-668 (1991)). It is a regular feature of normal animal development and is the fate of a substantial fraction of the cells produced in most animals. In vertebrates, for example, it regulates the numbers of neurons, eliminates undesirable types of lymphocytes, disposes cells that have finished their job (as when a tadpole loses its tail at metamorphosis), and helps to sculpt the shapes of developing organs (creating digits by doing away with the cells that lie between the digit rudiments in the limb bud, for example). Too little cell death can be as dangerous to the health of the multicellular organism as too much proliferation, and mutations which inhibit cell death by causing overexpression of bcl-2 *(vide infra)* have been implicated in the development of cancer.

In the nematode *Caenorhabditis elegans,* a genetic pathway of programmed cell death has been identified. Two genes, *ced-3* and *ced-4,* are essential for cells to undergo programmed cell death in *C. elegans* (Ellis, H.M., and Horvitz, H.R., *Cell,* 44:817-829 (1986)). Recessive mutations that eliminate the function of these two genes prevent normal programmed cell death during the development of *C. elegans.* One known vertebrate counterpart to *ced-3* protein is ICE. The overall amino acid identity between *ced-3* and ICE is 28%, with a region of 115 amino acids (residues 246-360 of *ced-3* and 164-278 of ICE) that shows the highest identity (43%). This region contains a conserved pentapeptide, QACRG (residues 356-360 of *ced-3*), which contains a cysteine known to be essential for ICE function. The similarity between *ced-3* and ICE suggests not only that *ced-3* might function as a cysteine protease but also that ICE might act as a vertebrate programmed cell death gene as well as function in cleaving proIL-1β into mature and active IL-1β.

Following the discovery of similarity between ICE and ced-3, several other homologous genes were cloned which are now part of the ICE/ced-3 family of cysteine proteases. They include Ich-1, CPP32, TX, ICErelIII, and LAP3 (Yuan, *et al., Cell,* 75: 641-652 (1993), Miura, *et al., Cell,* 75: 653-660 (1993), Wang, *et al., Cell,* 78:739-750 (1994), Fernades-Alnemri, *et al., J. Biol. Chem.,* 270: 15870-15876 (1995) and Duan, *et al., J. Biol. Chem.,* 271: 35013-35035 (1996)). The ICE/ced-3 family of cysteine proteases are believed to cleave several key cellular homeostasis proteins, and, therefore, promote apoptosis.

ICE mRNA has been detected in a variety of tissues, including peripheral blood monocytes, peripheral blood lymphocytes, peripheral blood neutrophils, resting and activated peripheral blood T lymphocytes, placenta, the B lymphoblastoid line CB23, and monocytic leukemia cell line THP-1 cells (Cerretti, D.P., *et al., Science,* 256:97-100 (1992)), suggesting that ICE may have another substrate in addition to pro-IL-1β. The substrate that ICE acts upon to cause cell death is presently unknown. One possibility is that it may be a vertebrate homolog of the *C. elegans* cell death gene *ced-4.* Alternatively, ICE might directly cause cell death by proteolytically cleaving proteins that are essential for cell viability.

ICE-LAP7 (FLICE), has recently been described Muzio M., *et al.,* "FLICE: A Novel FADD-Homologous ICE/CED-3-like Protease, is Recruited to the CD95 (Fas/APO-1) Death-Inducing Signaling Complex", *Cell,* 85:817-827 (1996). FLICE has recently been shown to induce apoptosis in several cell lines. When activated by cleavage with granzyme B, FLICE is capable of cleaving poly ADP-ribose polymerase (PARP), an enzyme which exhibits a characteristic cleavage during apoptosis. Furthermore, FLICE mutants with truncated or inactive protease domains inhibit FLICE-, Fas-, and TNFR1-mediated apoptosis.

FLICE and the related gene Mch-4 (Fernandes-Alnemri, *et al., Proc. Natl. Acad. Sci. USA,* 93: 7464-7469 (1996)) not only have homology to the ICE/ced-3 family of cysteine proteases but also have homology to the Death Effector Domains of the TNF-related genes such as FADD (Chinnaiyan, *et al., Cell,* 81: 505-512 (1995)). This allows them to function as adaptor molecules bridging the gap between a signal at the cell surface, binding of TNF to TNFR1 or FasL to Fas, and the proteases that execute the cell death program.

The mammalian gene *bcl-2,* has been found to protect immune cells called lymphocytes from cell suicide. Also, *crmA,* a cow pox virus gene protein product inhibits ICE's protease activity.

Clearly, there is a need for factors that are useful for inducing apoptosis for therapeutic purposes, for example, as an antiviral agent, an anti-tumor agent and to control embryonic development and tissue homeostasis. There is also a need for factors that are useful for preventing apoptosis for therapeutic purposes, for example, for treating ischemic injury such as stroke, myocardial infarction and reperfusion injury, for treating neurodegenerative disorders such as Alzheimer's disease, Parkinson's disease and amyotrophic lateral sclerosis; osteoporosis and osteoarthritis, polycystic kidney disease, chronic degenerative liver disease, AIDS and aplastic anemia. There is a need, therefore, for identification and characterization of such factors that are related to ICE/ced-3 proteases, and which can play a role in preventing, ameliorating or correcting dysfunctions or diseases.

### SUMMARY OF THE INVENTION

This invention relates to a newly identified protein, termed herein FLICE Inhibitor-1 (FIN-1). Thus, the present invention provides novel FIN-1 polynucleotides and polypeptides; variants and derivatives of the polynucleotides and polypeptides; processes for making the polynucleotides and the polypeptides, and their variants and derivatives; agonists and antagonists of the polypeptides; and uses of the polynucleotides, polypeptides, variants, derivatives, agonists and antagonists.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### BRIEF DESCRIPTION OF THE DRAWINGS

The following drawings depict certain embodiments of the invention. They are illustrative only and do not limit the invention otherwise disclosed herein.

Fig. 1A-1C shows the cDNA sequence (SEQ ID NO:1) and the deduced amino acid sequence of human FIN-1 (SEQ ID NO:2).

Fig. 2 shows the similarities between the DEDs of FIN-1 (amino acids 1-73 and amino acids 90 - 169 of SEQ ID NO:2, respectively) and the DEDs of FADD (SEQ ID NO:7), FLICE (SEQ ID NO:8 and 9, respectively), Mch-4 (SEQ ID NO: 10 and 11, respectively) and Pea-15 (SEQ ID NO:12). Also shown is a consensus sequence (SEQ ID NO:13).

Fig. 3A shows the homology between FIN-1 DED1 (amino acids 3-73 of SEQ ID NO:2) and FLICE DED1 (amino acids 4-81 of SEQ ID NO:8).

Fig. 3B shows the homology between FIN-1 DED2 (amino acids 93-169 of SEQ ID NO:2) and FLICE DED2 (SEQ ID NO:9).

Fig. 4 shows the similarity between the cysteine protease domains of CPP-32 (SEQ ID NO:3), ICE-LAP3 (SEQ ID NO:4), FLICE (SEQ ID NO:5), Mch-4 (SEQ ID NO:6), and FIN-1 (amino acids 170-480 of SEQ ID NO:2), and a consensus sequence [SEQ ID NO:14]. SEQ ID NO:15 is the consensus sequence of the active site of the ICE-ced-3 family.

Fig. 5 shows the homology between the FIN-1 (amino acids 184-478 of SEQ ID NO:2) and FLICE (amino acids 2-300 of SEQ ID NO:5) cysteine protease domains.

### GLOSSARY

The following illustrative explanations are provided to facilitate understanding of certain terms used frequently herein, particularly in the examples. The explanations are provided as a convenience and are not meant to limit the invention.

"Digestion" of DNA refers to catalytic cleavage of a DNA with an enzyme such as, but not limited to, a restriction enzyme that acts only at certain sequences in the DNA. The various restriction enzymes referred to herein are commercially available and their reaction conditions, cofactors and other requirements for use are known and routine to the skilled artisan.

For analytical purposes, typically, 1 microgram of plasmid or DNA fragment is digested with about 2 units of enzyme in about 20 microliters of reaction buffer. For the purpose of isolating DNA fragments for plasmid construction, typically 5 to 50 micrograms of DNA are digested with 20 to 250 units of enzyme in proportionately larger volumes.

Appropriate buffers and substrate amounts for particular restriction enzymes are described in standard laboratory manuals, such as those referenced below, and are specified by commercial suppliers.

Incubation times of about 1 hour at 37°C are ordinarily used, but conditions may vary in accordance with standard procedures, the supplier's instructions and the particulars of the reaction. After digestion, reactions may be analyzed, and fragments may be purified by electrophoresis through an agarose or polyacrylamide gel, using well known methods that are routine for those skilled in the art.

"Genetic element" generally means a polynucleotide comprising a region that encodes a polypeptide or a region that regulates replication, transcription, translation or other processes important to expression of the polypeptide in a host cell, or a polynucleotide comprising both a region that encodes a polypeptide and a region operably linked thereto that regulates expression.

Genetic elements may be comprised within a vector that replicates as an episomal element; that is, as a molecule physically independent of the host cell genome. They may be comprised within mini-chromosomes, such as those that arise during amplification of transfected DNA by methotrexate selection in eukaryotic cells. Genetic elements also may be comprised within a host cell genome, not in their natural state but, rather, following manipulation such as isolation, cloning and introduction into a host cell in the form of purified DNA or in a vector, among others.

"Isolated" means altered "by the hand of man" from its natural state; i.e., that, if it occurs in nature, it has been changed or removed from its original environment, or both. For example, a naturally occurring polynucleotide or a polypeptide naturally present in a living animal in its natural state is not "isolated", but the same polynucleotide or polypeptide separated from the coexisting materials of its natural state is "isolated", as the term is employed herein. For example, with respect to polynucleotides, the term isolated means that it is separated from the chromosome and cell in which it naturally occurs.

As part of or following isolation, such polynucleotides can be joined to other polynucleotides such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the polynucleotides and polypeptides may occur in a composition, such as a media, formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

"Ligation" refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook *et al.,* Molecular cloning, A Laboratory Manual, 2nd ed.; Cold Spring Harbor Laboratory Press, Cold Spring Harbor, New York, 1989, hereinafter referred to as Sambrook *et al.*

"Oligonucleotide(s)" refers to relatively short polynucleotides. Often the term refers to single-stranded deoxyribonucleotides, but it can refer as well to single- or double-stranded ribonucleotides, RNA:DNA hybrids and double-stranded DNAs, among others.

Oligonucleotides, such as single-stranded DNA probe oligonucleotides, often are synthesized by chemical methods, such as those implemented on automated oligonucleotide synthesizers. However, oligonucleotides can be made by a variety of other methods, including in vitro recombinant DNA-mediated techniques and by expression of DNAs in cells and organisms.

Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. Where ligation of such oligonucleotides is desired, a phosphate can be added by standard techniques, such as those that employ a kinase and ATP.

The 3' end of a chemically synthesized oligonucleotide generally has a free hydroxyl group and, in the presence of a ligase, such as T4 DNA ligase, will readily form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

"Plasmids" are genetic elements that are stably inherited without being a part of the chromosome of their host cell. They may be comprised of DNA or RNA and may be linear or circular. Plasmids code for molecules that ensure their replication and stable inheritance during cell replication and may encode products of considerable medical, agricultural and environmental importance. For example, they code for toxins that greatly increase the virulence of pathogenic bacteria. They can also encode genes that confer resistance to antibiotics. Plasmids are widely used in molecular biology as vectors used to clone and express recombinant genes. Plasmids generally are designated herein by a lower case "p" preceded and/or followed by capital letters and/or numbers, in accordance with standard naming conventions that are familiar to those of skill in the art. Starting plasmids disclosed herein are either commercially available, publicly available, or can be constructed from available plasmids by routine application of well known, published procedures. Many plasmids and other cloning and expression vectors that can be used in accordance with the present invention are well known and readily available to those of skill in the art. Moreover, those of skill may readily construct any number of other plasmids suitable for use in the invention. The properties, construction and use of such plasmids, as well as other vectors, in the present invention will be readily apparent to those of skill from the present disclosure.

"Polynucleotide(s)" generally refers to any polyribonucleotide or polydeoxribonucleotide, which may be unmodified RNA or DNA or modified RNA or DNA. Thus, for instance, polynucleotides as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single-and double-stranded regions. In addition, polynucleotide, as used herein, refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term polynucleotide also includes DNAs or RNAs as described above that contain one or more modified bases. Thus, DNAs or RNAs with backbones modified for stability or for other reasons are polynucleotides, as that term is intended herein. Moreover, DNAs or RNAs comprising unusual bases, such as inosine, or modified bases, such as tritylated bases, to name just two examples, are polynucleotides, as the term is used herein. It will be appreciated that a great variety of modifications have been made to DNA and RNA that serve many useful purposes known to those of skill in the art. The term polynucleotide, as it is employed herein, embraces such chemically, enzymatically or metabolically modified forms of polynucleotides, as well as the chemical forms of DNA and RNA characteristic of viruses and cells, including inter alia simple and complex cells.

"Polypeptides", as used herein, includes all polypeptides as described below. The basic structure of polypeptides is well known and has been described in innumerable textbooks and other publications in the art. In this context, the term is used herein to refer to any peptide or protein comprising two or more amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types.

It will be appreciated that polypeptides often contain amino acids other than the 20 amino acids commonly referred to as the 20 naturally occurring amino acids, and that many amino acids, including the terminal amino acids, may be modified in a given polypeptide, either by natural processes, such as processing and other post-translational modifications, or by chemical modification techniques which are well known to the art. Even the common modifications that occur naturally in polypeptides are too numerous to list exhaustively here, but they are well described in basic texts and in more detailed monographs, as well as in a voluminous research literature, and thus are well known to those of skill in the art. Known modifications which may be present in polypeptides of the present invention include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. Such modifications are well known to those of skill and have been described in great detail in the scientific literature. Several particularly common modifications including glycosylation, lipid attachment, sulfation, gamma-carboxylation of glutamic acid residues, hydroxylation and ADP-ribosylation are described in most basic texts such as Proteins - Structure and Molecular Properties, 2nd Ed., T. E. Creighton, W. H. Freeman and Company, New York, 1993. Detailed reviews are also available on this subject. *See, e.g.,* Wold, F., Posttranslational Protein Modifications: Perspectives and Prospects, pgs. 1-12 in Posttranslational Covalent Modification of Proteins, B. C. Johnson, ed., Academic Press, New York, 1983; Seifter *et al.,* "Analysis for protein modifications and nonprotein cofactors", *Meth. Enzymol.,* 182:626-646 (1990) and Rattan *et al.,* "Protein Synthesis: Posttranslational Modifications and Aging", *Ann. N.Y. Acad. Sci.,* 663: 48-62 (1992).

It will be appreciated, as is well known and as noted above, that polypeptides are not always entirely linear. For instance, polypeptides may be branched as a result of ubiquitination, and they may be circular, with or without branching, generally as a result of posttranslation events, including natural processing event and events brought about by human manipulation which do not occur naturally. Circular, branched and branched circular polypeptides may be synthesized by non-translation natural processes and by entirely synthetic methods, as well.

Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in E. coli, prior to processing, almost invariably will be N-formylmethionine.

The modifications that occur in a polypeptide often will be a function of how it is made. For polypeptides made by expressing a cloned gene in a host, for instance, the nature and extent of the modifications in large part will be determined by the host cell's posttranslational modification capacity and the modification signals present in the polypeptide amino acid sequence. For instance, as is well known, glycosylation often does not occur in bacterial hosts such as *E. coli.* Accordingly, when glycosylation is desired, a polypeptide should be expressed in a glycosylating host, generally a eukaryotic cell. Insect cells often carry out the same posttranslational glycosylations as mammalian cells and, for this reason, insect cell expression systems have been developed to express efficiently mammalian proteins having the native patterns of glycosylation, inter alia. Similar considerations apply to other modifications.

It will be appreciated that the same type of modification may be present in the same or varying degrees at several sites in a given polypeptide. Also, a given polypeptide may contain many types of modifications.

In general, as used herein, the term polypeptide encompasses all such modifications, particularly those that are present in polypeptides synthesized by expressing a polynucleotide in a host cell.

"Variant(s)" of polynucleotides or polypeptides, as the term is used herein, are polynucleotides or polypeptides that differ from a reference polynucleotide or polypeptide, respectively. Variants in this sense are described below and elsewhere in the present disclosure in greater detail.

Variants include polynucleotides that differ in nucleotide sequence from another, reference polynucleotide. Generally, differences are limited so that the nucleotide sequences of the reference and the variant are closely similar overall and, in many regions, identical.

As noted below, changes in the nucleotide sequence of the variant may be silent. That is, they may not alter the amino acids encoded by the polynucleotide. Where alterations are limited to silent changes of this type, a variant will encode a polypeptide with the same amino acid sequence as the reference. As also noted below, changes in the nucleotide sequence of the variant may alter the amino acid sequence of a polypeptide encoded by the reference polynucleotide. Such nucleotide changes may result in amino acid substitutions, additions, deletions, fusions and truncations in the polypeptide encoded by the reference sequence, as discussed below.

Variants also include polypeptides that differ in amino acid sequence from another, reference polypeptide. Generally, differences are limited so that the sequences of the reference and the variant are closely similar overall and, in many regions, identical.

A variant and reference polypeptide may differ in amino acid sequence by one or more substitutions, additions, deletions, fusions and truncations, which may be present in any combination.

"Fusion protein" as the term is used herein, is a protein encoded by two, often unrelated, fused genes or fragments thereof. EP-A-O 464 533 (Canadian counterpart 2045869) discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties (EP-A 0232 262). On the other hand, for some uses it would be desirable to be able to delete the Fc part after the fusion protein has been expressed, detected and purified. Accordingly, it may be desirable to link the components of the fusion protein with a chemically or enzymatically cleavable linking region. This is the case when the Fc portion proves to be a hindrance to use in therapy and diagnosis, for example, when the fusion protein is to be used as an antigen for immunizations. In drug discovery, for example, human proteins, such as, shIL5-a have been fused with Fc portions for use in high-throughput screening assays to identify antagonists of hIL-5. See, D. Bennett *et al., Journal of Molecular Recognition,* 8:52-58 (1995); and K. Johanson *et al., J. Biol. Chem.,* 270(16):9459-9471 (1995).

Thus, this invention also relates to genetically engineered soluble fusion proteins comprised of human FIN-1, or a portion thereof, and of various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. In one embodiment, the Fc part can be removed simply by incorporation of a cleavage sequence which can be cleaved with blood clotting factor Xa. This invention further relates to processes for the preparation of these fusion proteins by genetic engineering, and to the use thereof for diagnosis and therapy. Yet a further aspect of the invention relates to polynucleotides encoding such fusion proteins.

Membrane bound proteins are particularly useful in the formation of fusion proteins. Such proteins are generally characterized as possessing three distinct structural regions, an extracellular domain, a transmembrane domain, and a cytoplasmic domain. This invention contemplates the use of one or more of these regions as components of a fusion protein. Examples of such fusion protein technology can be found in WO94/29458 and WO94/22914.

"Binding molecules" (or otherwise called "interaction molecules" or "receptor component factors") refer to molecules, including receptors, that specifically bind to or interact with polypeptides of the present invention. Such binding molecules are a part of the present invention. Binding molecules may also be non-naturally occurring, such as antibodies and antibody-derived reagents that bind specifically to polypeptides of the invention.

As known in the art, "similarity" between two polypeptides is determined by comparing the amino acid sequence and its conserved amino acid substitutes of one polypeptide to the sequence of a second polypeptide. Moreover, also known in the art is "identity", which means the degree of sequence relatedness between two polypeptide or two polynucleotide sequences as determined by the identity of the match between two strings of such sequences. Both identity and similarity can be readily calculated (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). There exist a number of methods to measure identity and similarity between two polynucleotide or polypeptide sequences, and the terms "identity" and "similarity" are well known to skilled artisans (Carillo, H., and Lipton, D., *SIAM J. Applied Math.,* 48:1073 (1988)). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J. Applied Math.,* 48:1073 (1988). Preferred methods to determine identity are designed to give the largest match between the two sequences tested. Methods to determine identity and similarity are also codified in computer programs. Preferred computer program methods to determine identity and similarity between two sequences include, but are not limited to, GCS program package (Devereux, J., et *al., Nucl. Acids Res.,* 12(1):387 (1984)), BLAST, FAST (Atschul, S.F. *et al., J. Molec. Biol.,* 215:403 (1990).

### DESCRIPTION OF THE INVENTION

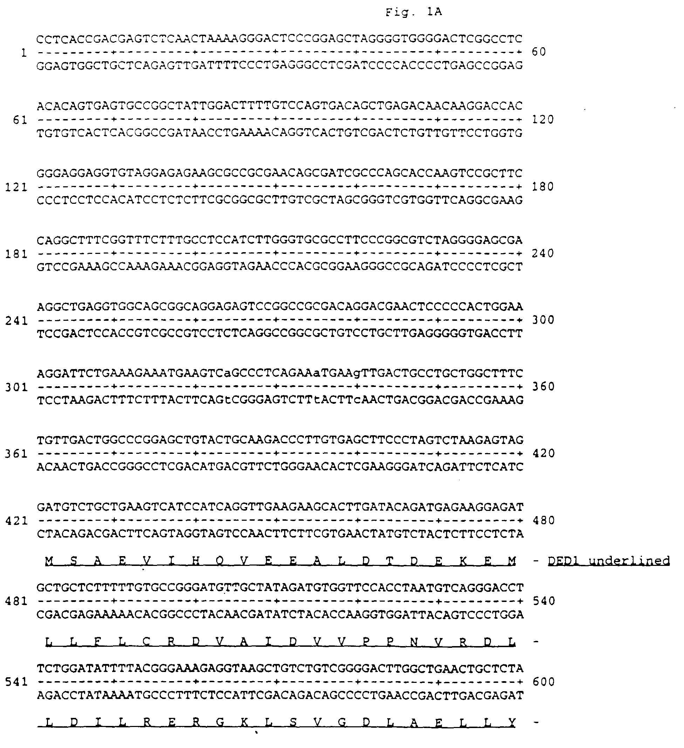

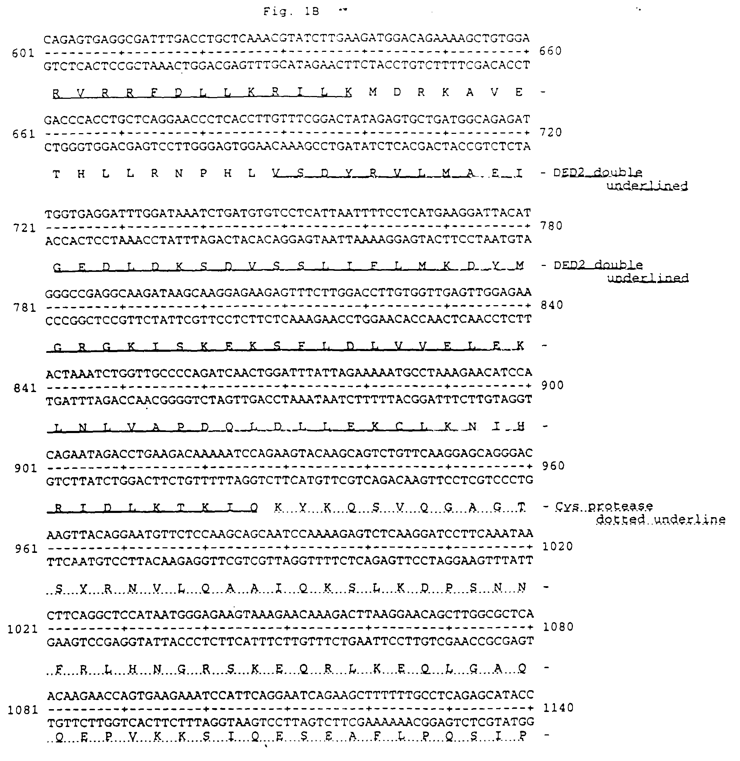

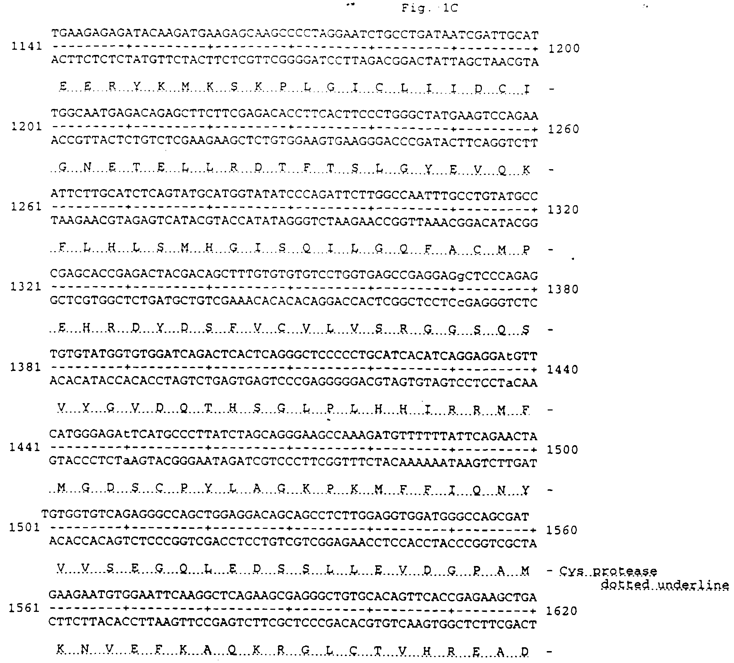

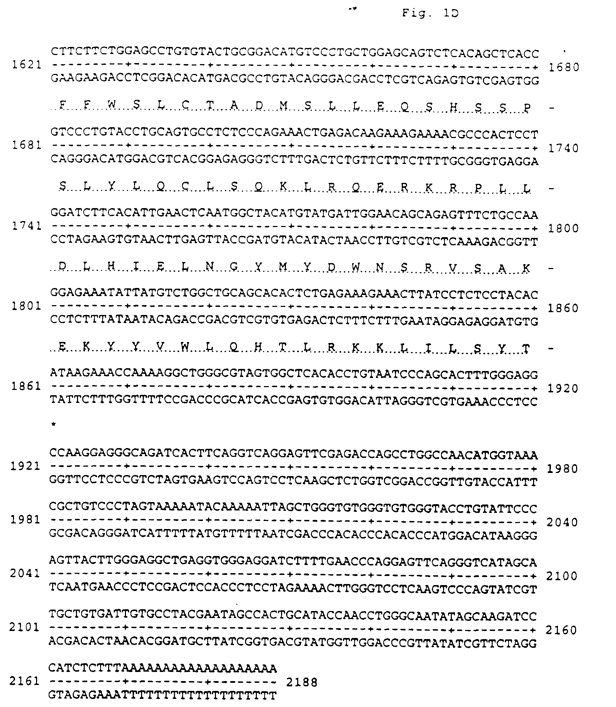

The present invention relates to novel human FIN-1 polypeptides and polynucleotides, among other things, as described in greater detail below. In particular, the invention relates to polypeptides and polynucleotides of a novel human FIN-1, which is related by amino acid sequence homology to ICE-LAP proteins and proteins with eeath effector domains such as FADD, FLICE and Mch-4. The invention relates especially to human FIN-1 having the nucleotide and amino acid sequences set out in Figure 1 and in SEQ ID NO:1 and 2.

### Polynucleotides

In accordance with one aspect of the present invention, there are provided isolated polynucleotides which encode the human FIN-1 polypeptide having the deduced amino acid sequence of Figure 1.

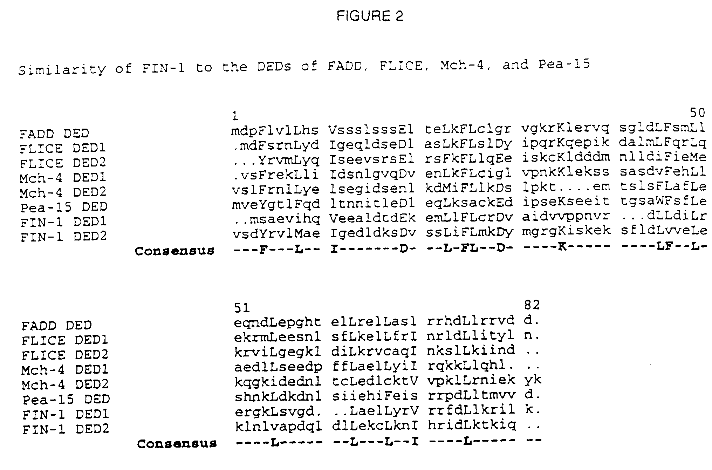

Using the information provided herein, such as the polynucleotide sequence set out in Figure 1, a polynucleotide of the present invention encoding human FIN-1 may be obtained using standard cloning and screening procedures. Illustrative of the invention, the polynucleotide set out in Figure 1 was discovered in a cDNA library derived from bone marrow cells. Using expressed sequence tag (EST) analysis [Adams, M.D., *et al., Science,* 252:1651-1656 (1991); Adams, M.D. *et al., Nature,* 355:632-634 (1992); Adams, M.D., *et al., Nature,* 377: Supp:3-174 (1995)] partial length clones have been identified from cerebellum, osteoclastoma, HL60 treated with PMA, T cell lymphoma, induced epithelium, HOS osteoblasts and greater omentum. Human FIN-1 is structurally related to other proteins of the ICE-LAP family, as shown by the results of sequencing the cDNA sequence set out in Figure 1, SEQ ID NO: 1. FIN-1 contains an open reading frame beginning at nucleotide 422 of Figure 1 (SEQ ID NO:1), encoding a protein of approximately 480 amino acids. The open reading frame of human FIN-1 encodes two DEDs. An alignment of these novel DEDs with those of FADD, FLICE, Mch4 and Pea-15 demonstrates that the first and second DEDs of FIN-1 exhibit similarity to the DED consensus sequence at 14 and 15 out of 17 residues, respectively. This is roughly equivalent to the level of similarity of the other members of the family to the consensus (see Figure 2). FIN-1 has several potential phosphorylation sites and a potential N-linked glycosylation site.

Polynucleotides of the present invention may be in the form of RNA, such as mRNA, or in the form of DNA, including, for instance, cDNA and genomic DNA obtained by cloning or produced by chemical synthetic techniques or by a combination thereof. The DNA may be double-stranded or single-stranded. Single-stranded DNA may be the coding strand, also known as the sense strand, or it may be the non-coding strand, also referred to as the anti-sense strand.

The coding sequence which encodes the polypeptide may be identical to the coding sequence of the polynucleotide shown in Figure 1, SEQ ID NO: 1. It also may be a polynucleotide with a different sequence, which, as a result of the redundancy (degeneracy) of the genetic code, also encodes the polypeptide of Figure 1, SEQ ID NO: 2.

Polynucleotides of the present invention which encode the polypeptide of Figure 1 may include, but are not limited to, the coding sequence for the mature polypeptide, by itself; the coding sequence for the mature polypeptide and additional coding sequences; and the coding sequence of the mature polypeptide, with or without the aforementioned additional coding sequences, together with additional, non-coding sequences. Examples of additional coding sequence include, but are not limited to, sequences encoding a leader or secretory sequence, such as a pre-, or pro- or prepro- protein sequence. Examples of additional non-coding sequences include, but are not limited to, introns and non-coding 5' and 3' sequences, such as the transcribed, non-translated sequences that play a role in transcription, and mRNA processing, including splicing and polyadenylation signals, for example, for ribosome binding and stability of mRNA. Coding sequences which provide additional functionalities may also be incorporated into the polypeptide. Thus, for instance, the polypeptide may be fused to a marker sequence, such as a peptide, which facilitates purification of the fused polypeptide. In certain preferred embodiments of this aspect of the invention, the marker sequence is a hexa-histidine peptide, such as the HA tag provided in the pQE vector (Qiagen, Inc.). As described in Gentz et *al., Proc. Natl. Acad. Sci. USA,* 86:821-824 (1989), for instance, hexa-histidine provides for convenient purification of the fusion protein. The HA tag corresponds to an epitope derived of influenza hemagglutinin protein, which has been described by Wilson *et al., Cell,* 37:767 (1984), for instance. Many other such tags are commercially available.

In accordance with the foregoing, the term "polynucleotide encoding a polypeptide" as used herein encompasses polynucleotides which include, by virtue of the redundancy of the genetic code, any sequence encoding a polypeptide of the present invention, particularly the human FIN-1 having the amino acid sequence set out in Figure 1, SEQ ID NO:2. The term also encompasses polynucleotides that include a single continuous region or discontinuous regions encoding the polypeptide (for example, interrupted by introns) together with additional regions, that also may contain coding and/or non-coding sequences.

The present invention further relates to variants of the herein above described polynucleotides which encode fragments, analogs and derivatives of the polypeptide having the deduced amino acid sequence of Figure 1. A variant of the polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms.

Among variants in this regard are variants that differ from the aforementioned polynucleotides by nucleotide substitutions, deletions or additions. The substitutions, deletions or additions may involve one or more nucleotides. The variants may be altered in coding or non-coding regions or both. Alterations in the coding regions may produce conservative or non-conservative amino acid substitutions, deletions or additions.

Among the particularly preferred embodiments of the invention in this regard are polynucleotides encoding polypeptides having the amino acid sequence of human FIN-1 set out in Figure 1; variants, analogs, derivatives and fragments thereof, and fragments of the variants, analogs and derivatives.

Further particularly preferred in this regard are polynucleotides encoding human FIN-1 variants, analogs, derivatives and fragments, and variants, analogs and derivatives of the fragments, which have the amino acid sequence of the human FIN-1 polypeptide of Figure 1 in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the human FIN-1. Also especially preferred in this regard are conservative substitutions. Most highly preferred are polynucleotides encoding polypeptides having the amino acid sequence of Figure 1, without substitutions.

Further preferred embodiments of the invention are polynucleotides that are at least 70% identical to a polynucleotide encoding the human FIN-1 polypeptide having the amino acid sequence set out in Figure 1, and polynucleotides which are complementary to such polynucleotides. More preferred are polynucleotides that comprise a region that is at least 80% identical to a polynucleotide encoding the human FIN-1 polypeptide and polynucleotides complementary thereto. Polynucleotides at least 90% identical to the same are particularly preferred, and those with at least 95% are more particularly preferred. Furthermore, those with at least 97% are highly preferred and those with at least 98-99% are more highly preferred, with at least 99% being the most preferred.

Particularly preferred embodiments in this respect, moreover, are polynucleotides which encode polypeptides which retain substantially the same biological function or activity as the mature polypeptide encoded by the cDNA of Figure 1.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

As discussed additionally herein regarding polynucleotide assays of the invention, for instance, polynucleotides of the invention as discussed above, may be used as hybridization probes for cDNA and genomic DNA, to isolate full-length cDNAs and genomic clones encoding human FIN-1 and to isolate cDNA and genomic clones of other genes that have a high sequence similarity to the human FIN-1 gene. Such probes generally will comprise at least 15 nucleotides. Preferably, such probes will have at least 30 nucleotides and may have at least 50 nucleotides. Particularly preferred probes will range between 30 and 50 nucleotides.

For example, the coding region of the human FIN-1 gene may be isolated by screening using the known DNA sequence to synthesize an oligonucleotide probe. A labeled oligonucleotide having a sequence complementary to that of a gene of the present invention is then used to screen a library of human cDNA, genomic DNA or mRNA to determine the members of the library to which the probe hybridizes.

The polynucleotides and polypeptides of the present invention may be employed as research reagents and materials for discovery of treatments and diagnostics to human disease, as further discussed herein relating to polynucleotide assays.

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may facilitate protein trafficking, may prolong or shorten protein half-life or may facilitate manipulation of a protein for assay or production, among other things. As generally is the case *in situ,* the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In sum, a polynucleotide of the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Polypeptides

The present invention further relates to a human FIN-1 polypeptide which has the deduced amino acid sequence of Figure 1, SEQ ID NO: 2.

The invention also relates to fragments, analogs and derivatives of these polypeptides. The terms "fragment," "derivative" and "analog" when referring to the polypeptide of Figure 1, mean a polypeptide which retains essentially the same biological function or activity as such polypeptide, i.e. functions as a FIN-1. Thus, an analog includes, for example, a proprotein which can be activated by cleavage of the proprotein portion to produce an active mature polypeptide.

The polypeptide of the present invention may be a recombinant polypeptide, a natural polypeptide or a synthetic polypeptide. In certain preferred embodiments, it is a recombinant polypeptide.

The fragment, derivative or analog of the polypeptide of Figure 1 may be: (i) one in which one or more of the amino acid residues are substituted with a conserved or non-conserved amino acid residue (preferably a conserved amino acid residue) and such substituted amino acid residue may or may not be one encoded by the genetic code; (ii) one in which one or more of the amino acid residues includes a substituent group; (iii) one in which the mature polypeptide is fused with another compound, such as a compound to increase the half-life of the polypeptide (for example, polyethylene glycol); or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

Among the particularly preferred embodiments of the invention in this regard are polypeptides having the amino acid sequence of human FIN-1 set out in Figure 1 as SEQ ID NO:2, variants, analogs, derivatives and fragments thereof, and variants, analogs and derivatives of the fragments. Further particularly preferred embodiments of the invention in this regard are polypeptides having the amino acid sequence of human FIN-1, variants, analogs, derivatives and fragments thereof, and variants, analogs and derivatives of the fragments which retain the activity/function of this protein.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe and Tyr.

Further particularly preferred in this regard are variants, analogs, derivatives and fragments, and variants, analogs and derivatives of the fragments, having the amino acid sequence of the human FIN-1 polypeptide of Figure 1, in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the protein. Also especially preferred in this regard are conservative substitutions. Most highly preferred are polypeptides having the amino acid sequence of Figure 1, SEQ ID NO:2, without substitutions.

The polypeptides and polynucleotides of the present invention are preferably provided in an isolated form, and preferably are purified to homogeneity.

The polypeptides of the present invention include the polypeptide of SEQ ID NO:2 (in particular the mature polypeptide) as well as polypeptides which have at least 70% identity to the polypeptide of SEQ ID NO:2 and more preferably at least 90% similarity (more preferably at least 90% identity) to the polypeptide of SEQ ID NO:2 and still more preferably at least 95% similarity (still more preferably at least 95% identity) to the polypeptide of SEQ ID NO:2 and also include portions of such polypeptides with such portion of the polypeptide generally containing at least 30 amino acids and more preferably at least 50 amino acids.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention. Fragments may be "free-standing," i.e., not part of or fused to other amino acids or polypeptides, or they may be comprised within a larger polypeptide of which they form a part or region. When comprised within a larger polypeptide, the presently discussed fragments most preferably form a single continuous region. However, several fragments may be comprised within a single larger polypeptide. For instance, certain preferred embodiments relate to a fragment of a human FIN-1 polypeptide of the present comprised within a precursor polypeptide designed for expression in a host and having heterologous pre- and pro-polypeptide regions fused to the amino terminus of the human FIN-1 fragment and an additional region fused to the carboxyl terminus of the fragment. Therefore, fragments in one aspect of the meaning intended herein, refers to the portion or portions of a fusion polypeptide or fusion protein derived from human FIN-1.

As representative examples of polypeptide fragments of the invention, there may be mentioned those which have from about 5-15, 10-20, 15-40, 30-55, 41-75, 41-80, 41-90, 50-100, 75-100, 90-115, 100-125, and 110-113 amino acids in length.

In this context "about" includes the particularly recited range and ranges larger or smaller by several, a few, 5, 4, 3, 2 or 1 amino acid residues at either extreme or at both extremes. For instance, about 40-90 amino acids in this context means a polypeptide fragment of 40 plus or minus several, a few, 5, 4, 3, 2 or 1 amino acid residues to 90 plus or minus several, a few, 5, 4, 3, 2 or 1 amino acid residues, i.e., ranges as broad as 40 minus several amino acids to 90 plus several amino acids to as narrow as 40 plus several amino acids to 90 minus several amino acids. Highly preferred in this regard are the recited ranges plus or minus as many as 5 amino acids at either or at both extremes. Particularly highly preferred are the recited ranges plus or minus as many as 3 amino acids at either or at both the recited extremes. Especially particularly highly preferred are ranges plus or minus 1 amino acid at either or at both extremes or the recited ranges with no additions or deletions. Most highly preferred of all in this regard are fragments from about 5-15, 10-20, 15-40, 30-55, 41-75, 41-80, 41-90, 50-100, 75-100, 90-115, 100-125, and 110-113 amino acids long.

Among especially preferred fragments of the invention are truncation mutants of human FIN-1. Truncation mutants include human FIN-1 polypeptides having the amino acid sequence of Figure 1, or of variants or derivatives thereof, except for deletion of a continuous series of residues (that is, a continuous region, part or portion) that includes the amino terminus, or a continuous series of residues that includes the carboxyl terminus or, as in double truncation mutants, deletion of two continuous series of residues, one including the amino terminus and one including the carboxyl terminus. Fragments having the size ranges set out above are also preferred embodiments of truncation fragments, which are especially preferred among fragments generally.

Also preferred in this aspect of the invention are fragments characterized by structural or functional attributes of human FIN-1. Preferred embodiments of the invention in this regard include fragments that comprise alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of human FIN-1.

Among highly preferred fragments in this regard are those that comprise regions of human FIN-1 that combine several structural features, such as several of the features set out above. Such regions may be comprised within a larger polypeptide or may be by themselves a preferred fragment of the present invention, as discussed above. It will be appreciated that the term "about" as used in this paragraph has the meaning set out above regarding fragments in general.

Further preferred regions are those that mediate activities of the FIN-1 protein. Most highly preferred in this regard are fragments that have a chemical, biological or other activity of human FIN-1, including those with a similar activity or an improved activity, or with a decreased undesirable activity. Highly preferred in this regard are fragments that contain regions that are homologs in sequence, or in position, or in both sequence and position to active regions of related polypeptides, such as human FIN-1. Among particularly preferred fragments in these regards are truncation mutants, as discussed above, or fragments comprising cytoplasmic, transmembrane or extracellular domains.

It will be appreciated that the invention also relates to, among others, polynucleotides encoding the aforementioned fragments, polynucleotides that hybridize to polynucleotides encoding the fragments, particularly those that hybridize under stringent conditions, and polynucleotides, such as PCR primers, for amplifying polynucleotides that encode the fragments. In these regards, preferred polynucleotides are those that correspond to the preferred fragments, as discussed above.

### Vectors, host cells, expression

The present invention also relates to vectors which contain polynucleotides of the present invention, host cells which are genetically engineered with vectors of the invention and the production of polypeptides of the invention by recombinant techniques.

Host cells can be genetically engineered to incorporate polynucleotides and express polypeptides of the present invention. For instance, polynucleotides may be introduced into host cells using well known techniques of infection, transduction, transfection, transvection and transformation. The polynucleotides may be introduced alone or with other polynucleotides. Such other polynucleotides may be introduced independently, co-introduced or introduced joined to the polynucleotides of the invention.

Thus, for instance, polynucleotides of the invention may be transfected into host cells with another, separate polynucleotide encoding a selectable marker, using standard techniques for co-transfection and selection in, for instance, mammalian cells. In this case the polynucleotides generally will be stably incorporated into the host cell genome.

Alternatively, the polynucleotides may be joined to a vector containing a selectable marker for propagation in a host. The vector construct may be introduced into host cells by the aforementioned techniques. Generally, a plasmid vector is introduced as DNA in a precipitate, such as a calcium phosphate precipitate, or in a complex with a charged lipid. Electroporation may also be used to introduce polynucleotides into a host. If the vector is a virus, it may be packaged in vitro or introduced into a packaging cell and the packaged virus may be transduced into cells. A wide variety of techniques suitable for making polynucleotides and for introducing polynucleotides into cells in accordance with this aspect of the invention are well known and routine to those of skill in the art. Such techniques are reviewed at length in Sambrook *et al.,* which is merely illustrative of the many laboratory manuals that detail these techniques.

In accordance with this aspect of the invention the vector may be, for example, a plasmid vector, a single-or double-stranded phage vector, or a single- or double-stranded RNA or DNA viral vector. Such vectors may be introduced into cells as polynucleotides, preferably DNA, by well known techniques for introducing DNA and RNA into cells. The vectors, in the case of phage and viral vectors may also be and preferably are introduced into cells as packaged or encapsidated virus by well known techniques for infection and transduction. Viral vectors may be replication competent or replication defective. In the latter case, viral propagation generally will occur only in complementing host cells.

Preferred among vectors, in certain respects, are those for expression of polynucleotides and polypeptides of the present invention. Generally, such vectors comprise cis-acting control regions effective for expression in a host operatively linked to the polynucleotide to be expressed. Appropriate trans-acting factors are either supplied by the host, supplied by a complementing vector or supplied by the vector itself upon introduction into the host.

In certain preferred embodiments in this regard, the vectors provide for specific expression. Such specific expression may be inducible expression or expression only in certain types of cells or both inducible and cell-specific expression. Particularly preferred among inducible vectors are vectors that can be induced to express a protein by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

The engineered host cells can be cultured in conventional nutrient media, which may be modified as appropriate for, inter alia, activating promoters, selecting transformants or amplifying genes. Culture conditions, such as temperature, pH and the like, previously used with the host cell selected for expression, generally will be suitable for expression of polypeptides of the present invention as will be apparent to those of skill in the art.

A great variety of expression vectors can be used to express a polypeptide of the invention. Such vectors include chromosomal, episomal and virus-derived vectors e.g., vectors derived from bacterial plasmids, bacteriophages, yeast episomes, yeast chromosomal elements, and viruses such as baculoviruses, papova viruses, SV40, vaccinia viruses, adenoviruses, fowl pox viruses, pseudorabies viruses and retroviruses, and vectors derived from combinations thereof, such as those derived from plasmid and bacteriophage genetic elements, cosmids and phagemids. Generally, any vector suitable to maintain, propagate or express polynucleotides to produce a polypeptide in a host may be used for expression in this regard.

The appropriate DNA sequence may be inserted into the vector by any of a variety of well-known and routine techniques. In general, a DNA sequence for expression is joined to an expression vector by cleaving the DNA sequence and the expression vector with one or more restriction endonucleases and then joining the restriction fragments together using T4 DNA ligase. Procedures for restriction and ligation that can be used to this end are well known and routine to those of skill. Suitable procedures in this regard, and for constructing expression vectors using alternative techniques, which also are well known and routine to those skilled in the art, are set forth in great detail in Sambrook *et al.*

The DNA sequence in the expression vector is operatively linked to appropriate expression control sequence(s), including, for instance, a promoter to direct mRNA transcription. Representatives of such promoters include the phage lambda PL promoter, the E. coli lac, trp and tac promoters, the SV40 early and late promoters and promoters of retroviral LTRs, to name just a few of the well-known promoters. It will be understood that numerous other promoters useful in this aspect of the invention are well known and may be routinely employed by those of skill in the manner illustrated by the discussion and the examples herein.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate, as well as engender, expression. Generally, in accordance with many commonly practiced procedures, such regions will operate by controlling transcription. Examples include repressor binding sites and enhancers, among others.

Vectors for propagation and expression generally will include selectable markers. Selectable marker genes provide a phenotypic trait for selection of transformed host cells. Preferred markers include, but are not limited to, dihydrofolate reductase or neomycin resistance for eukaryotic cell culture, and tetracycline or ampicillin resistance genes for culturing *E. coli* and other bacteria. Such markers may also be suitable for amplification. Alternatively, the vectors may contain additional markers for this purpose.

The vector containing a selected polynucleotide sequence as described elsewhere herein, as well as an appropriate promoter, and other appropriate control sequences, may be introduced into an appropriate host using a variety of well known techniques suitable for expression therein of a desired polypeptide. Representative examples of appropriate hosts include bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS and Bowes melanoma cells; and plant cells. Hosts of a great variety of expression constructs are well known, and those of skill will be enabled by the present disclosure to routinely select a host for expressing a polypeptide in accordance with this aspect of the present invention.

More particularly, the present invention also includes recombinant constructs, such as expression constructs, comprising one or more of the sequences described above. The constructs comprise a vector, such as a plasmid or viral vector, into which such a sequence of the invention has been inserted. The sequence may be inserted in a forward or reverse orientation. In certain preferred embodiments in this regard, the construct further comprises regulatory sequences, including, for example, a promoter, operably linked to the sequence. Large numbers of suitable vectors and promoters are known to those of skill in the art, and there are many commercially available vectors suitable for use in the present invention.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. These vectors are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide of the invention in a host may be used in this aspect of the invention.

Promoter regions can be selected from any desired gene using vectors that contain a reporter transcription unit lacking a promoter region, such as a chloramphenicol acetyl transferase ("CAT") transcription unit, downstream of a restriction site or sites for introducing a candidate promoter fragment; i.e., a fragment that may contain a promoter. As is well known, introduction into the vector of a promoter-containing fragment at the restriction site upstream of the CAT gene engenders production of CAT activity, which can be detected by standard CAT assays. Vectors suitable to this end are well known and readily available. Two examples of such vectors include pKK232-8 and pCM7. Thus, promoters for expression of polynucleotides of the present invention include not only well known and readily available promoters, but also promoters that may be readily obtained by the foregoing technique, using a reporter gene.

Among known bacterial promoters suitable for expression of polynucleotides and polypeptides in accordance with the present invention are the E. coli lacI and lacz promoters, the T3 and T7 promoters, the gpt promoter, the lambda PR, PL promoters and the trp promoter.

Among known eukaryotic promoters suitable in this regard are the CMV immediate early promoter, the HSV thymidine kinase promoter, the early and late SV40 promoters, the promoters of retroviral LTRs, such as those of the Rous Sarcoma Virus("RSV"), and metallothionein promoters, such as the mouse metallothionein-I promoter.

Selection of appropriate vectors and promoters for expression in a host cell is a well known procedure and the requisite techniques for construction of expression vectors, introduction of the vector into the host and expression in the host are routine skills in the art.

The present invention also relates to host cells containing the above-described constructs. The host cell can be a higher eukaryotic cell, such as a mammalian cell, a lower eukaryotic cell, such as a yeast cell, or a prokaryotic cell, such as a bacterial cell.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals.

Constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook *et al.*

Generally, recombinant expression vectors will include origins of replication, a promoter derived from a highly-expressed gene to direct transcription of a downstream structural sequence, and a selectable marker to permit isolation of vector containing cells following exposure to the vector. Among suitable promoters are those derived from the genes that encode glycolytic enzymes such as 3-phosphoglycerate kinase ("PGK"), alpha-factor, acid phosphatase, and heat shock proteins, among others. Selectable markers include the ampicillin resistance gene of E. coli and the trpl gene of S. *cerevisiae.*

Transcription of DNA encoding the polypeptides of the present invention by higher eukaryotes may be increased by inserting an enhancer sequence into the vector. Enhancers are cis-acting elements of DNA, usually from about 10 to 300 bp, that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

A polynucleotide of the invention encoding the heterologous structural sequence of a polypeptide of the invention generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed. Generally, there will be no other open reading frames that begin with an initiation codon, usually AUG, and lie between the ribosome binding site and the initiation codon. Also, generally, there will be a translation stop codon at the end of the polypeptide and a polyadenylation signal and transcription termination signal appropriately disposed at the 3' end of the transcribed region.

Appropriate secretion signals may be incorporated into the expressed polypeptide for secretion of the translated protein into the lumen of the endoplasmic reticulum, the periplasmic space or the extracellular environment. The signals may be endogenous to the polypeptide or heterologous.

The polypeptide may be expressed in a modified form, such as a fusion protein, and may include not only secretion signals but also additional heterologous functional regions. Thus, for example, a region of additional amino acids, particularly charged amino acids, may be added to the N-terminus of the polypeptide to improve stability and persistence in the host cell during purification or subsequent handling and storage. A region may also be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art.

Suitable prokaryotic hosts for propagation, maintenance or expression of polynucleotides and polypeptides in accordance with the invention include *Escherichia coli, Bacillus subtilis* and *Salmonella typhimurium.* Various species of Pseudomonas, Streptomyces, and Staphylococcus are also suitable hosts in this regard. Moreover, many other hosts also known to those of skill may be employed in this regard.

As a representative but non-limiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). In these vectors, the pBR322 "backbone" sections are combined with an appropriate promoter and the structural sequence to be expressed.

Following transformation of a suitable host strain, the host strain is grown to an appropriate cell density. Where the selected promoter is inducible, it is induced by appropriate means (e.g., temperature shift or exposure to chemical inducer) and cells are cultured for an additional period. Cells typically then are harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents. Such methods are well known to those skilled in the art.

Various mammalian cell culture systems can be employed for expression, as well. Examples of mammalian expression systems include the C127, 3T3, CHO, HeLa, human kidney 293 and BHK cell lines. and the COS-7 line of monkey kidney fibroblasts, described by Gluzman *et al., Cell,* 23:175 (1981).

Mammalian expression vectors will comprise an origin of replication, a suitable promoter and enhancer, and any necessary ribosome binding sites, polyadenylation sites, splice donor and acceptor sites, transcriptional termination sequences, and 5' flanking non-transcribed sequences that are necessary for expression. In certain preferred embodiments, DNA sequences derived from the SV40 splice sites and the SV40 polyadenylation sites are used for required non-transcribed genetic elements.

The human FIN-1 polypeptide can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Well known techniques for refolding proteins may be employed to regenerate active conformation when the polypeptide is denatured during isolation and or purification.

Polypeptides of the present invention include naturally purified polypeptides, polypeptides produced by chemical synthetic procedures, and polypeptides produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or non-glycosylated. In addition, polypeptides of the invention may include an initial modified methionine residue, in some cases as a result of host-mediated processes.

Human FIN-1 polynucleotides and polypeptides may be used in accordance with the present invention for a variety of applications, particularly those that make use of the chemical and biological properties of the protein. Additional applications relate to diagnosis and to treatment of disorders of cells, tissues and organisms. These aspects of the invention are illustrated further by the following discussion.

### Polynucleotide assays

This invention is also related to the use of human FIN-1 polynucleotides to detect complementary polynucleotides for use, for example, as a diagnostic reagent. Detection of a mutated form of human FIN-1 associated with a dysfunction will provide a diagnostic tool that can add to or define diagnosis of a disease or susceptibility to a disease which results from under-expression, over-expression or altered expression of human FIN-1. Individuals carrying mutations in the human FIN-1 gene may be detected at the DNA level by a variety of techniques. Nucleic acids for diagnosis may be obtained from a patient's cells, such as from blood, urine, saliva, tissue biopsy or autopsy material. The genomic DNA may be used directly for detection or may be amplified enzymatically by using PCR prior to analysis (Saiki *et al., Nature,* 324:163-166 (1986). RNA or cDNA may also be used in similar fashion. As an example, PCR primers complementary to the nucleic acid encoding human FIN-1 can be used to identify and analyze human FIN-1 expression and mutations. For example, deletions and insertions can be detected by a change in size of the amplified product in comparison to the normal genotype. Point mutations can be identified by hybridizing amplified DNA to radiolabeled FIN-1 RNA or, radiolabeled FIN-1 antisense DNA sequences. Perfectly matched sequences can be distinguished from mismatched duplexes by RNase A digestion or by differences in melting temperatures.

Sequence differences between a reference gene and genes having mutations may also be revealed by direct DNA sequencing. In addition, cloned DNA segments may be employed as probes to detect specific DNA segments. The sensitivity of such methods can be greatly enhanced by appropriate use of PCR or other amplification methods. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alterations in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (*see, e.g.,* Myers *et al., Science,* 230:1242 (1985).

Sequence changes at specific locations may also be revealed by nuclease protection assays, such as RNase and S1 protection or the chemical cleavage method (*e.g.,* Cotton *et al., Proc. Natl. Acad. Sci. USA,* 85:4397-4401 (1985). Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP") and Southern blotting of genomic DNA.

In accordance with a further aspect of the invention, there is provided a process for diagnosing or determining a susceptibility to viral diseases and tumors, among others. In addition, there is provided a method for controlling embryonic development and providing tissue homeostasis. A mutation in the human FIN-1 gene may be indicative of a susceptibility to viral diseases and tumors, among others; and the nucleic acid sequences described above may be employed in an assay for ascertaining such susceptibility. Thus, for example, the assay may be employed to determine a mutation in a human FIN-1 gene, as herein described, such as a deletion, truncation, insertion, frame shift, etc., with such mutation being indicative of a susceptibility to a hyperproliferative disease, among others.

The invention provides a process for diagnosing diseases, particularly, viral diseases and tumors. In addition, FIN-1 sequences may be useful in diagnosis of ischemic injury such as stroke, myocardial infarction and reperfusion injury, for diagnosis of neurodegenerative disorders such as Alzheimer's disease, Parkinson's disease and amyotrophic lateral sclerosis; osteoporosis and osteoarthritis, polycystic kidney disease, chronic degenerative liver disease, AIDS and aplastic anemia. This process comprises determining from a sample derived from a patient an abnormally decreased or increased level of expression of polynucleotide having the sequence of Figure 1, SEQ ID NO:1. Decreased or increased expression of polynucleotide can be measured using any of the methods well known in the art for the quantitation of polynucleotides, such as, for example, PCR, RT-PCR, RNase protection, Northern blotting and other hybridization methods.

In addition to more conventional gel-electrophoresis and DNA sequencing, mutations can also be detected by in situ analysis.

### Chromosome assays

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with gene associated disease.

Briefly, sequences can be mapped to chromosomes by preparing PCR primers (preferably 15-25 bp) from the cDNA. Computer analysis of the 3' untranslated region is used to rapidly select primers that do not span more than one exon in the genomic DNA, because primers that span more than one exon could complicate the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can be used similarly to map to the chromosome include in situ hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNAs as short as 50 to 60 bases. For a review of this technique, see Verma *et al.,* Chromosomes: A Manual of Basic Techniques, Pergamon Press, New York, 1988.

As an example of how this technique is performed, human FIN-1 DNA is digested and purified with QIAEX II DNA purification kit (QIAGEN, Inc., Chatsworth, CA) and ligated to Super Cos1 cosmid vector (STRATAGENE, La Jolla, CA). DNA is purified using Qiagen Plasmid Purification Kit (QIAGEN Inc., Chatsworth, CA) and 1 mg is labeled by nick translation in the presence of Biotin-dATP using BioNick Labeling Kit (GibcoBRL, Life Technologies Inc., Gaithersburg, MD) . Biotinylation is detected with GENE-TECT Detection System (CLONTECH Laboratories, Inc. Palo Alto, CA). In situ hybridization is performed on slides using ONCOR Light Hybridization Kit (ONCOR, Gaithersberg, MD) to detect single copy sequences on metaphase chromosomes. Peripheral blood of normal donors is cultured for three days in RPMI 1640 supplemented with 20% FCS, 3% PHA and penicillin/streptomycin, synchronized with 10-7 M methotrexate for 17 hours, and washed twice with unsupplemented RPMI. Cells are then incubated with 10-3 M thymidine for 7 hours. The cells are arrested in metaphase after a 20 minute incubation with colcemid (0.5 µg/ml) followed by hypotonic lysis in 75 mM KCl for 15 minutes at 37°C. Cell pellets are then spun out and fixed in Carnoy's fixative (3:1 methanol/acetic acid).

Metaphase spreads are prepared by adding a drop of the suspension onto slides and air drying the suspension. Hybridization is performed by adding 100 ng of probe suspended in 10 ml of hybridization mix (50% formamide, 2xSSC, 1% dextran sulfate) with blocking human placental DNA (1 µg/ml). Probe mixture is denatured for 10 minutes in a 70°C water bath and incubated for 1 hour at 37°C, before placement on a prewarmed (37°C) slide, previously denatured in 70% formamide/2xSSC at 70°C, dehydrated in ethanol series, and chilled to 4°C.

Slides are incubated for 16 hours at 37°C in a humidified chamber. Slides are washed in 50% formamide/2xSSC for 10 minutes at 41°C and 2xSSC for 7 minutes at 37°C. Hybridization probe is detected by incubation of the slides with FITC-Avidin (ONCOR, Gaithersberg, MD), according to the manufacturer's protocol. Chromosomes are counterstained with propridium iodine suspended in mounting medium. Slides are visualized using a Leitz ORTHOPLAN 2-epifluorescence microscope and five computer images are taken using a Imagenetics Computer and MacIntosh printer.

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

It is then necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes assuming 1 megabase mapping resolution and one gene per 20 kb.

### Polypeptide assays

The present invention also relates to diagnostic assays for detecting levels of human FIN-1 protein in cells and tissues. Such assays may be quantitative or qualitative, Thus, for instance, a diagnostic assay in accordance with the invention for detecting over-expression of human FIN-1 protein compared to normal control tissue samples may be used to detect the presence of viral infections or tumors. Assay techniques that can be used to determine levels of a protein, such as a human FIN-1 protein of the present invention, in a sample derived from a host are well-known to those of skill in the art. Such assay methods include radioimmunoassays, competitive-binding assays, Western Blot analysis and enzyme linked immunosorbent assays (ELISAs). Among these, ELISAs are frequently preferred. An ELISA assay initially comprises preparing an antibody specific to human FIN-1, preferably a monoclonal antibody. In addition a reporter antibody generally is prepared which binds to the monoclonal antibody. The reporter antibody is attached to a detectable reagent such as a radioactive, fluorescent or enzymatic reagent, in this example, horseradish peroxidase enzyme.

To carry out an ELISA, a sample is removed from a host and incubated on a solid support, e.g. a polystyrene dish, that binds the proteins in the sample. Any free protein binding sites on the dish are then covered by incubating with a non-specific protein such as bovine serum albumin. The monoclonal antibody is then incubated in the dish during which time the monoclonal antibodies attach to any human FIN-1 proteins attached to the polystyrene dish. Unbound monoclonal antibody is washed out with buffer. The reporter antibody linked to horseradish peroxidase is placed in the dish resulting in binding of the reporter antibody to any monoclonal antibody bound to FIN-1 protein. Unattached reporter antibody is then washed out. Reagents for peroxidase activity, including a colorimetric substrate are then added to the dish. Immobilized peroxidase, linked to human FIN-1 through the primary and secondary antibodies, produces a colored reaction product. The amount of color developed in a given time period indicates the amount of human FIN-1 protein present in the sample. Quantitative results typically are obtained by reference to a standard curve.

A competition assay may also be employed wherein antibodies specific to human FIN-1 attached to a solid support and labeled human FIN-1 and a sample derived from the host are passed over the solid support. The amount of detected label attached to the solid support can be correlated to a quantity of human FIN-1 in the sample.

### Antibodies

The polypeptides, their fragments or other derivatives, or analogs thereof, or cells expressing them can also be used as immunogens to produce antibodies thereto. These antibodies can be, for example, polyclonal or monoclonal antibodies. The present invention also includes chimeric, single chain, and humanized antibodies, as well as Fab fragments, or the product of a Fab expression library. Various procedures known in the art may be used for the production of such antibodies and fragments.

Antibodies generated against polypeptides corresponding to a sequence of the present invention can be obtained by various means well known to those of skill in the art. For example, in one embodiment, the polypeptide is directly injected into an animal, preferably a nonhuman. The antibody so obtained will then bind the polypeptide itself. In this embodiment, even a sequence encoding only a fragment of the polypeptide can be used to generate antibodies binding the whole native polypeptide. Such antibodies can then be used to isolate the polypeptide from tissues expressing that polypeptide.

For preparation of monoclonal antibodies, any technique which provides antibodies produced by continuous cell line cultures can be used. Examples include the hybridoma technique (Kohler, G. and Milstein, C., Nature, 1975, 256:495-497), the trioma technique, the human B-cell hybridoma technique (Kozbor *et al., Immunology Today,* 4:72 (1983)) and the EBV-hybridoma technique (Cole *et al.,* Monoclonal Antibodies and Cancer Therapy, pg. 77-96, Alan R. Liss, Inc., 1985).

Techniques described for the production of single chain antibodies (U.S. Patent No. 4,946,778) can also be adapted to produce single chain antibodies to immunogenic polypeptide products of this invention. Also, transgenic mice, or other organisms including other mammals, may be used to express humanized antibodies to immunogenic polypeptide products of this invention.

The above-described antibodies may be employed to isolate or to identify clones expressing the polypeptide or to purify the polypeptide of the present invention by attachment of the antibody to a solid support for isolation and/or purification by affinity chromatography.

Antibodies against human FIN-1 may also be employed to inhibit viral infection and cancers, particularly those associated with solid tumors, among others.

### Binding molecules and assays

Human FIN-1 can also be used to isolate proteins which interact with it; this interaction can be a target for interference. Inhibitors of protein-protein interactions between human FIN-1 and other factors could lead to the development of pharmaceutical agents for the modulation of human FIN-1 activity.

Thus, this invention also provides a method for identification of binding molecules to human FIN-1. Genes encoding proteins for binding molecules to human FIN-1 can be identified by numerous methods known to those of skill in the art, for example, ligand panning, co-immunoprecipitation and the yeast two-hybrid system. Such methods are described in many laboratory manuals such as, for instance, Coligan *et al., Current Protocols in Immunology,* 1, Chapter 5, 1991.

For example, the yeast two-hybrid system provides methods for detecting the interaction between a first test protein and a second test protein, in vivo, using reconstitution of the activity of a transcriptional activator. The method is disclosed in U.S. Patent No. 5,283,173; reagents are available from Clontech and Stratagene. Briefly, human FIN-1 cDNA is fused to a Gal4 transcription factor DNA binding domain and expressed in yeast cells. cDNA library members obtained from cells of interest are fused to a transactivation domain of Gal4. cDNA clones which express proteins which can interact with human FIN-1 will lead to reconstitution of Gal4 activity and transactivation of expression of a reporter gene such as Gal4-lacZ.

An alternative method involves screening of lambda gtll, lambda ZAP (Stratagene) or equivalent cDNA expression libraries with recombinant human FIN-1. Recombinant human FIN-1 protein or fragments thereof are fused to small peptide tags such as FLAG, HSV or GST. The peptide tags can possess convenient phosphorylation sites for a kinase such as heart muscle creatine kinase or they can be biotinylated. Recombinant human FIN-1 can be phosphorylated with 32[P] or used unlabeled and detected with streptavidin or antibodies against the tags. Lambda gtll cDNA expression libraries are made from cells of interest and are incubated with the recombinant human FIN-1, washed and cDNA clones which interact with human FIN-1 isolated. Such methods are routinely used by skilled artisans. See, e.g., Sambrook *et al.*

Another method is the screening of a mammalian expression library. In this method, cDNAs are cloned into a vector between a mammalian promoter and polyadenylation site and transiently transfected in COS or 293 cells. Forty-eight hours later, the binding protein is detected by incubation of fixed and permabilized cells with labeled human FIN-1. In a preferred embodiment, the human FIN-1 is iodinated, and any bound human FIN-1 is detected by autoradiography. See Sims *et al., Science,* 241:585-589 (1988) and McMahan *et al., EMBO J.,* 10:2821-2832 (1991). In this manner, pools of cDNAs containing the cDNA encoding the binding protein of interest can be selected and the cDNA of interest can be isolated by further subdivision of each pool followed by cycles cf transient transfection, binding and autoradiography. Alternatively, the cDNA of interest can be isolated by transfecting the entire cDNA library into mammalian cells and panning the cells on a dish containing human FIN-1 bound to the plate. Cells which attach after washing are lysed and the plasmid DNA isolated, amplified in bacteria, and the cycle of transfection and panning repeated until a single cDNA clone is obtained. *See*, Seed *et al., Proc. Natl. Acad. Sci. USA*, 84:3365 (1987) and Aruffo *et al., EMBO J.,* 6:3313 (1987). If the binding protein is secreted, its cDNA can be obtained by a similar pooling strategy once a binding or neutralizing assay has been established for assaying supernatants from transiently transfected cells. General methods for screening supernatants are disclosed in Wong *et al., Science,* 228:810-815 (1985).

Another method involves isolation of proteins interacting with human FIN-1 directly from cells. Fusion proteins of human FIN-1 with GST or small peptide tags are made and immobilized on beads. Biosynthetically labeled or unlabeled protein extracts from the cells of interest are prepared, incubated with the beads and washed with buffer. Proteins interacting with human FIN-1 are eluted specifically from the beads and analyzed by SDS-PAGE. Binding partner primary amino acid sequence data are obtained by microsequencing. Optionally, the cells can be treated with agents that induce a functional response such as tyrosine phosphorylation of cellular proteins. An example of such an agent would be a growth factor or cytokine such as interleukin-2.

Another method is immunoaffinity purification. Recombinant human FIN-1 is incubated with labeled or unlabeled cell extracts and immunoprecipitated with anti-human antibodies. The immunoprecipitate is recovered with protein A-Sepharose and analyzed by SDS-PAGE. Unlabelled proteins are labeled by biotinylation and detected on SDS gels with streptavidin. Binding partner proteins are analyzed by microsequencing. Further, standard biochemical purification steps known to those skilled in the art may be used prior to microsequencing.

Yet another alternative method involves screening of peptide libraries for binding partners. Recombinant tagged or labeled human FIN-1 is used to select peptides from a peptide or phosphopeptide library which interact with human FIN-1. Sequencing of the peptides leads to identification of consensus peptide sequences which might be found in interacting proteins.

### Agonists and antagonists - assays and molecules

The human FIN-1 of the present invention may be employed in a screening process for compounds which activate (agonists) or inhibit activation (antagonists) of this protein.

Examples of potential kinase antagonists include antibodies or, in some cases, oligonucleotides which bind to the protein but do not elicit a second messenger response such that the activity of the protein is prevented.

Potential antagonists also include proteins which are closely related to human FIN-1, i.e. a fragment of the protein, which have lost biological activity.

A potential antagonist also includes an antisense construct prepared through the use of antisense technology. Antisense technology can be used to control gene expression through triple-helix formation or antisense DNA or RNA, both methods of which are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee *et al., Nucl. Acids* *Res.,* 6:3073 (1979); Cooney *et al.,* Science, 241:456 (1988); and Dervan *et al., Science,* 251:1360 (1991), thereby preventing transcription and production of the human FIN-1. The antisense RNA oligonucleotide hybridizes to the mRNA in vivo and blocks translation of the mRNA molecule into the protein (antisense - see Okano, *J. Neurochem.,* 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA is expressed in vivo to inhibit production of human FIN-1.

Another potential antagonist is a small molecule which binds to the protein, making it inaccessible to ligands such that normal biological activity is prevented. Examples of small molecules include, but are not limited to, small peptides or peptide-like molecules.

Potential antagonists also include soluble forms of human FIN-1, e.g., fragments of the protein, which bind to ligands, thus preventing the ligand from interacting with human FIN-1.

It is desirable to find compounds and drugs which stimulate protein activity on the one hand and which can inhibit the function of FIN-1 on the other hand.

Antagonists for human FIN-1 may be employed for a variety of therapeutic and prophylactic purposes for viral infections and tumors, among others.

This invention additionally provides a method of treating an abnormal condition related to an excess of human FIN-1 activity which comprises administering to a subject an inhibitor compound (antagonist) as hereinabove described along with a pharmaceutically acceptable carrier in an amount effective to inhibit activation of the protein, or by inhibiting a second signal, and thereby alleviating the abnormal condition.

In general, agonists for human FIN-1 are employed for therapeutic and prophylactic purposes for such diseases or disorders where prevention of programmed cell death is preferred. The invention also provides a method of treating abnormal conditions related to an under-expression of human FIN-1 and its activity, which comprises administering to a subject a therapeutically effective amount of a compound which activates (agonist) the protein, to thereby alleviate the abnormal condition.

### Compositions and Kits

The soluble form of human FIN-1, and compounds which activate or inhibit such protein, may be employed in combination with a suitable pharmaceutical carrier. Such compositions comprise a therapeutically effective amount of the polypeptide or compound, and a pharmaceutically acceptable carrier or excipient. Such carriers include, but are not limited to, saline, buffered saline, dextrose, water, glycerol, ethanol, and combinations thereof. The formulation should suit the mode of administration. Selection of an appropriate carrier in accordance with the mode of administration is routinely performed by those skilled in the art.

The invention further relates to pharmaceutical packs and kits comprising one or more containers filled with one or more of the ingredients of the aforementioned compositions of the invention.

### Administration

Polypeptides and other compounds of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes, among others.

The pharmaceutical compositions generally are administered in an amount effective for treatment or prophylaxis of a specific indication or indications. In general, the compositions are administered in an amount of at least about 10 micrograms/kg body weight. In most cases they will be administered in an amount not in excess of about 8 mg/kg body weight per day. Preferably, in most cases, the administered dose is from about 10 micrograms/kg to about 1 mg/kg body weight, daily. It will be appreciated that optimum dosage will be determined by standard methods for each treatment modality and indication, taking into account the indication, its severity, route of administration, complicating conditions and the like.

### Gene therapy

Human FIN-1 polynucleotides, polypeptides, agonists and antagonists that are polypeptides may be employed in accordance with the present invention by expression of such polypeptides in treatment modalities often referred to as "gene therapy."

Thus, for example, cells from a patient may be engineered with a polynucleotide, such as a DNA or RNA, to encode a polypeptide ex vivo. The engineered cells can then be provided to a patient to be treated with the polypeptide. In this embodiment, cells may be engineered ex vivo, for example, by the use of a retroviral plasmid vector containing RNA encoding a polypeptide of the present invention. Such methods are well-known in the art and their use in the present invention will be apparent from the teachings herein.

Similarly, cells may be engineered in vivo for expression of a polypeptide in vivo by procedures known in the art. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a patient for engineering cells in vivo and expression of the polypeptide in vivo. These and other methods for administering a polypeptide of the present invention should be apparent to those skilled in the art from the teachings of the present invention.

Retroviruses from which the retroviral plasmid vectors herein above mentioned may be derived include, but are not limited to, Moloney Murine Leukemia Virus, Spleen Necrosis Virus, Rous Sarcoma Virus, Harvey Sarcoma Virus, Avian Leukosis Virus, Gibbon Ape Leukemia Virus, Human Immunodeficiency Virus, Adenovirus, Myeloproliferative Sarcoma Virus, and Mammary Tumor Virus. In a preferred embodiment, the retroviral plasmid vector is derived from Moloney Murine Leukemia Virus.

Such vectors will include one or more promoters for expressing the polypeptide. Suitable promoters which may be employed include, but are not limited to, the retroviral LTR; the SV40 promoter; and the human cytomegalovirus (CMV) promoter described in Miller *et al., Biotechniques,* 7:980-990 (1989). Cellular promoters such as eukaryotic cellular promoters including, but not limited to, the histone, RNA polymerase III, and β-actin promoters can also be used. Additional viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The nucleic acid sequence encoding the polypeptide of the present invention will be placed under the control of a suitable promoter. Suitable promoters which may be employed include, but are not limited to, adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs herein above described); the β-actin promoter; and human growth hormone promoters. The promoter may also be the native promoter which controls the gene encoding the polypeptide. The retroviral plasmid vector is employed to transduce packaging cell lines to form producer cell lines. Examples of packaging cells which may be transfected include, but are not limited to, the PE501, PA317, Y-2, Y-AM, PA12, T19-14X, VT-19-17-H2, YCRE, YCRIP, GP+E-86, GP+envAm12, and DAN cell lines as described in Miller, A., Human Gene Therapy, 1990, 1:5-14. The vector may be transduced into the packaging cells through any means known in the art. Such means include, but are not limited to, electroporation, the use of liposomes, and CaPO4 precipitation. In one alternative, the retroviral plasmid vector may be encapsulated into a liposome, or coupled to a lipid, and then administered to a host.

The producer cell line will generate infectious retroviral vector particles, which include the nucleic acid sequence(s) encoding the polypeptides. Such retroviral vector particles may then be employed to transduce eukaryotic cells, either in vitro or in vivo. The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

### EXAMPLES

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

Certain terms used herein are explained in the foregoing glossary.

All examples are carried out using standard techniques, which are well known and routine to those of skill in the art, except where otherwise described in detail. Routine molecular biology techniques of the following examples can be carried out as described in standard laboratory manuals, such as Sambrook *et al.*

### Example 1 - Isolation and Identification of FIN-1

Oligonucleotide specific to FIN-1 were designed based on a nucleotide sequence contig derived from multiple EST sequences. A FIN-1 RT-PCR product (including nucleotides 284-620 of the FIN-1 cDNA sequence of Fig. 1, SEQ ID NO:1) was generated from RNA of PMA-treated HL60 cells using these oligonucleotides, and the RT-PCR product was used to screen human bone marrow and anergic T-cell libraries.

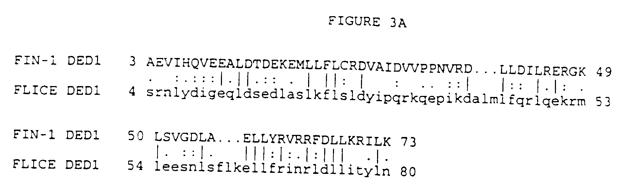

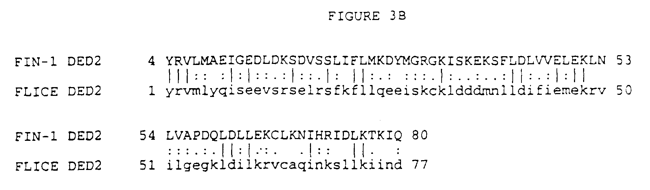

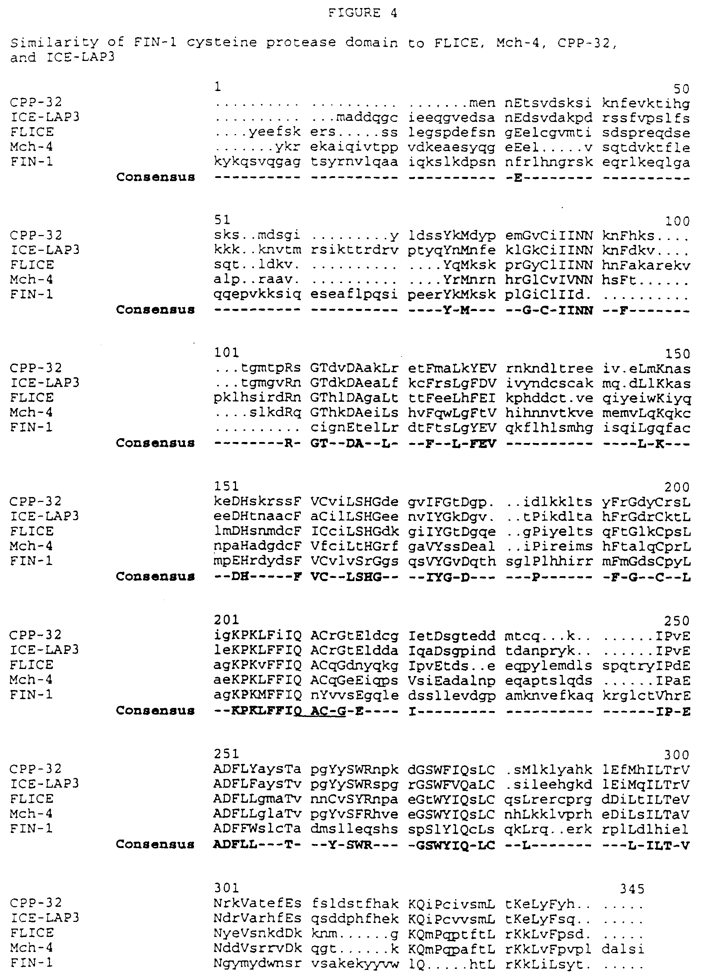

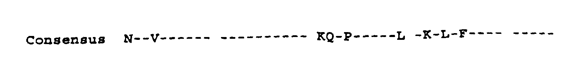

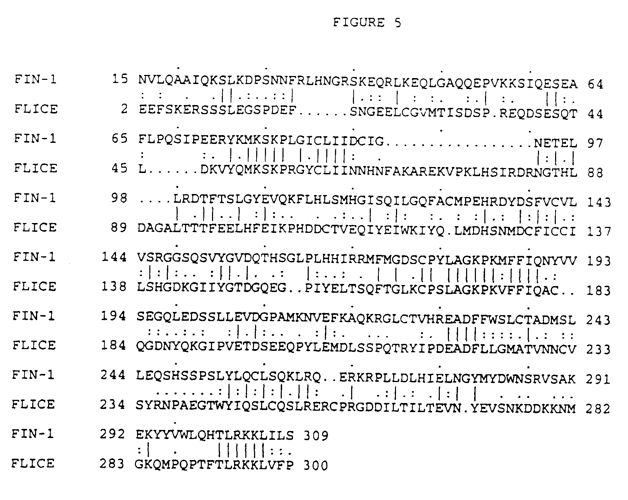

The sequence of 9 independent clones indicates that several alternatively spliced forms are produced. Figure 1 provides the cDNA sequence of one of these clones (SEQ ID NO:1), which encodes a 480 amino acid protein of 55 kD (SEQ ID NO:2) with two Death Effector Domains (DEDs) and a cysteine protease domain similar to that of FLICE and Mch-4 (see Figures 1, 2 and 4). An alignment of these novel DEDs with those of FADD, FLICE, Mch4 (a FLICE homologue), and Pea-15 demonstrates that the first and second DEDs of FIN-1 exhibit similarity to the DED consensus sequence at 14 and 15 out of 17 residues, respectively. This is roughly equivalent to the level of similarity of other members of the family to the consensus (Fig. 2). DED1, located within amino acids 1 to 73 of SEQ ID NO:2, is 30% identical and 56% similar to DED1 of FLICE (Fig. 3A). FIN-1 DED2 is located within amino acids 90 to 169 of SEQ ID NO:2 and is 27% identical and 66% similar to that of FLICE (Fig. 3B). The cysteine protease domain is located at amino acids 170-480. This cysteine protease domain does not contain the conserved QAC(R,Q)G (SEQ ID NO:15) active site motif (underlined in Figure 4), suggesting that this may be a dominant negative homologue of the ICE/ced-3 family. The cysteine protease domain of FIN-1 is 28% identical and 51% similar to that of FLICE (see Fig. 5).

### Example 2 - Identification of Ligands or Antagonists

The FIN-1 protein described above is desirably produced recombinant using the techniques described herein and used to screened for ligands or antagonists as follows.

The FIN-1 protein is utilized to screen compound banks, complex biological fluids, combinatorial organic and peptide libraries, etc. to identify activating ligands or antagonists. For example, the FIN-1 is employed to screen for (a) naturally occurring compounds which may be putative binding proteins for FIN-1; (b) non-mammalian, biologically active peptides for which there may be as yet undiscovered mammalian counterparts, (c) compounds not found in nature, but which appear to activate or interact with FIN-1 with unknown natural ligands and others.

Similarly, FIN-1 is screened against tissue extracts of human, and other mammalian, species, such as porcine tissue. Specifically such tissue extracts include lung, liver, gut, heart, kidney, adrenals, ischemic brain, plasma, urine and placenta. Initial extraction procedures focus on removal of bulk protein via acid or ethanol precipitation to bias the separation towards peptides and small molecules that account for a high percentage of known natural ligands of FIN-1. Subsequently milder extraction procedures are used to identify proteins. Extraction techniques employed in the formation of these tissue banks are known in the art.

### Example 3 - Expression of human FIN-1 for Gene Therapy

Fibroblasts are obtained from a subject by skin biopsy. The resulting tissue is placed in tissue-culture medium and separated into small pieces. Small chunks of the tissue are placed on a wet surface of a tissue culture flask; approximately ten pieces are placed in each flask. The flask is turned upside down, closed tight and left at room temperature overnight. After 24 hours at room temperature, the flask is inverted; the chunks of tissue remain fixed to the bottom of the flask; and fresh media (e.g., Ham's F12 media, with 10% FBS, penicillin and streptomycin) is added. The tissue is then incubated at 37°C for approximately one week. At this time, fresh media is added and subsequently changed every several days. After an additional two weeks in culture, a monolayer of fibroblasts emerges. The monolayer is trypsinized and scaled into larger flasks.

A vector for gene therapy (e.g., pMV-7 [Kirschmeier, P.T. *et al., DNA,* 7:219-25 (1988)] flanked by the long terminal repeats of the Moloney murine sarcoma virus, is digested with EcoRI and HindIII for cloning a fragment to be expression. The digested vector is treated with calf intestinal phosphatase to prevent self-ligation. The dephosphorylated, linear vector is fractionated on an agarose gel and purified, e.g., using glass beads.

FIN-1 capable of expressing active FIN-1 is isolated and amplified using PCR primers which correspond to the 5' and 3' end sequences, respectively. The ends of the fragment are modified, if necessary, for cloning into the vector. For instance, 5' overhanging ends may be treated with DNA polymerase to create blunt ends. 3' overhanging ends may be removed using S1 nuclease. Linkers may be ligated to blunt ends with T4 DNA ligase.

Equal quantities of the Moloney Murine Leukemia Virus linear backbone and the FIN-1 fragment are mixed together and joined using T4 DNA ligase. The ligation mixture is used to transform *E. coli* and the bacteria are then plated onto agar-containing kanamycin. Kanamycin phenotype and restriction analysis confirm that the vector has the properly inserted gene.

Packaging cells (amphotropic pA317 or GP+aml2 packaging cells) are grown in tissue culture to confluent density in Dulbecco's Modified Eagles Medium (DMEM) with 10% calf serum (CS), penicillin and streptomycin. The vector containing the FIN-1 gene is introduced into the packaging cells by standard techniques. Infectious viral particles containing the FIN-1 gene are collected from the packaging cells, which now are called producer cells.

Fresh media is added to the producer cells, and after an appropriate incubation period, media is harvested from the plates of confluent producer cells. The media, containing the infectious viral particles, is filtered through a MILLIPORE filter (Bedford, MA) to remove detached producer cells. The filtered media is then used to infect fibroblast cells. Media is removed from a sub-confluent plate of fibroblasts and quickly replaced with the filtered media. POLYBRENE (Aldrich Chemical Co., Milwaukee, WI) may be included in the media to facilitate transduction. After appropriate incubation, the media is removed and replaced with fresh media. If the titer of virus is high, then virtually all fibroblasts will be infected and no selection is required. If the titer is low, then it is necessary to use a retroviral vector that has a selectable marker, such as neo or his, to select out transduced cells for expansion.

Engineered fibroblasts may then be injected into rats, either alone or after having been grown to confluence on microcarrier beads such as CYTODEX 3 beads. The injected fibroblasts produce FIN-1 product, and the biological actions of the protein are conveyed to the host.

It will be clear that the invention may be practiced otherwise than as particularly described in the foregoing description and examples. Numerous modifications and variations of the present invention are possible in light of the above teachings and, therefore, are within the scope of the appended claims.

## Claims

1. An isolated polynucleotide comprising a member selected from the group consisting of:
(a) a polynucleotide having at least a 70% identity to a polynucleotide encoding a polypeptide comprising amino acids of SEQ ID NO: 2;
(b) a polynucleotide which by virtue of the redundancy of the genetic code, encodes the same amino acids of SEQ ID NO: 2;
(c) a polynucleotide which is complementary to the polynucleotide of (a) or (b); and
(d) a polynucleotide comprising at least 15 contiguous bases of the polynucleotide of (a), (b) or (c).

2. The polynucleotide of Claim 1 wherein the polynucleotide is DNA.

3. The polynucleotide of Claim 1 wherein the polynucleotide is RNA.

4. The polynucleotide of Claim 2 comprising nucleotides set forth in SEQ ID NO: 1.

5. The polynucleotide of Claim 2 which encodes a polypeptide comprising amino acids of SEQ ID NO: 2.

6. A vector comprising the DNA of Claim 2.

7. A host cell comprising the vector of Claim 6.

8. A process for producing a polypeptide comprising expressing from the host cell of Claim 7 a polypeptide encoded by said DNA.

9. A process for producing a cell which expresses a polypeptide comprising transforming or transfecting the cell with the vector of Claim 6 such that the cell expresses the polypeptide encoded by the human cDNA contained in the vector.

10. A polypeptide comprising an amino acid sequence which is at least 70% identical to the amino acid sequence of SEQ ID NO: 2.

11. A polypeptide comprising an amino acid sequence as set forth in SEQ ID NO: 2.

12. An agonist to the polypeptide of claim 10.

13. An antibody against the polypeptide of claim 10.

14. An antagonist to the polypeptide of claim 10.

15. A method for the treatment of a patient having need of FIN-1 comprising administering to the patient a therapeutically effective amount of the polypeptide of claim 10.

16. The method of Claim 15 wherein said therapeutically effective amount of the polypeptide is administered by providing to the patient DNA encoding said polypeptide and expressing said polypeptide in vivo.

17. A method for the treatment of a patient having need to inhibit FIN-1 polypeptide comprising administering to the patient a therapeutically effective amount of the antagonist of Claim 14.

18. A process for diagnosing a disease or a susceptibility to a disease related to expression of the polypeptide of claim 10 comprising determining a mutation in the nucleic acid sequence encoding said polypeptide.

19. A diagnostic process comprising analyzing for the presence of the polypeptide of claim 10 in a sample derived from a host.

20. A method for identifying compounds which bind to and activate or inhibit a receptor for the polypeptide of claim 10 comprising:
contacting a cell expressing on the surface thereof a receptor for the polypeptide, said receptor being associated with a second component capable of providing a detectable signal in response to the binding of a compound to said receptor, with a compound to be screened under conditions to permit binding to the receptor; and
determining whether the compound binds to and activates or inhibits the receptor by detecting the presence or absence of a signal generated from the interaction of the compound with the receptor.
